(19) Europäisches Patentamt / European Patent Office / Office européen des brevets

(11) EP 3 796 001 B1

(12) **EUROPEAN PATENT SPECIFICATION**

(45) Date of publication and mention of the grant of the patent:
**09.08.2023 Bulletin 2023/32**

(21) Application number: **19804276.4**

(22) Date of filing: **17.05.2019**

(51) International Patent Classification (IPC):
***G01N 33/574*** *(2006.01)* ***C07K 14/42*** *(2006.01)*
***C07K 16/18*** *(2006.01)* ***C12N 15/09*** *(2006.01)*

(52) Cooperative Patent Classification (CPC):
**G01N 33/57434;** C07K 14/42; C07K 16/18; C12N 15/09; G01N 2400/38; G01N 2440/38

(86) International application number:
**PCT/JP2019/019707**

(87) International publication number:
**WO 2019/221279 (21.11.2019 Gazette 2019/47)**

(54) **METHOD FOR DETERMINING PROSTATE CARCINOMA**

VERFAHREN ZUR BESTIMMUNG VON PROSTATAKARZINOMEN

PROCÉDÉ DE DÉTERMINATION DU CARCINOME DE LA PROSTATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.05.2018 JP 2018096252**

(43) Date of publication of application:
**24.03.2021 Bulletin 2021/12**

(73) Proprietors:
- **FUJIFILM Corporation**
  **Tokyo 106-8620 (JP)**
- **HIROSAKI UNIVERSITY**
  **Hirosaki-shi, Aomori 036-8560 (JP)**

(72) Inventors:
- **OHYAMA, Chikara**
  **Hirosaki-shi, Aomori 036-8560 (JP)**
- **YONEYAMA, Tohru**
  **Hirosaki-shi, Aomori 036-8560 (JP)**
- **TOBISAWA, Yuki**
  **Hirosaki-shi, Aomori 036-8560 (JP)**
- **ISHIKAWA, Tomokazu**
  **Amagasaki-shi, Hyogo 661-0963 (JP)**
- **DATE, Mutsuhiro**
  **Amagasaki-shi, Hyogo 661-0963 (JP)**
- **NAKAMURA, Kenji**
  **Osaka-shi, Osaka 540-8605 (JP)**

(74) Representative: **Winter, Brandl - Partnerschaft mbB**
**Alois-Steinecker-Straße 22**
**85354 Freising (DE)**

(56) References cited:
**EP-A1- 2 908 136 WO-A1-2017/130578**
**JP-B2- 4 514 919**

- **TOMOKAZU ISHIKAWA ET AL: "An Automated Micro-Total Immunoassay System for Measuring Cancer-Associated 2,3-linked Sialyl N-Glycan-Carrying Prostate-Specific Antigen May Improve the Accuracy of Prostate Cancer Diagnosis", INTERNATIONAL JOURNAL OF MOLECULAR SCIENCES, vol. 18, no. 2, 22 February 2017 (2017-02-22), page 470, XP055503642, DOI: 10.3390/ijms18020470**
- **KAZUTO ITO ET AL: "The diagnostic accuracy of the age-adjusted and prostate volume-adjusted biopsy method in males with prostate specific antigen levels of 4.1-10.0 ng/mL", CANCER, AMERICAN CANCER SOCIETY , PHILADELPHIA , PA, US, vol. 95, no. 10, 31 October 2002 (2002-10-31), pages 2112-2119, XP071036601, ISSN: 0008-543X, DOI: 10.1002/CNCR.10941**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- OYAMA, CHIKARA et al.: "Development and clinical application of glycan-targeted biomarkers for prostate cancer", The Official Journal of Japanese Society of Laboratory Medicin e, vol. 65, no. 2, 25 February 2017 (2017-02-25), pages 210-217, XP009524281,
- UNO, HIROMI: "Clinical significance of PSA-density in differential diagnosis between bph and early stages prostate cancer", The Japanese Journal of Urology, vol. 86, no. 12, December 1995 (1995-12), pages 1776-1783, XP055653241,

## Description

### Technical Field

The present invention relates to a novel method for determining prostate carcinoma.

### Background Art

Prostate carcinoma has the highest prevalence of malignant tumors in Western men, and the number of patients with prostate carcinoma has been rapidly increasing in Japan in recent years. According to 2015 statistics, prostate carcinoma has become the most prevalent cancer among Japanese men, surpassing gastric cancer.

The prostate specific antigen (hereinafter, abbreviated as "PSA") is a type of a glycoprotein produced in the prostate and specific to the prostate and having a molecular weight of about 30,000, and has an asparagine-linked (N-type) glycan. A PSA value is recognized as the most important tumor marker for determining prostate carcinoma in that the PSA value in blood increases in a case where an individual is affected with prostate carcinoma (Non-Patent Literature 1).

Most of PSA exists in blood as bound PSA that forms a complex by binding to a binding protein such as $\alpha$1-antichymotrypsin or $\alpha$2-macroglobulin (hereinafter, abbreviated as "bound PSA"). In addition, some PSA exists as a free form that does not form a complex (hereinafter, abbreviated as "free PSA").

The currently widely used method for diagnosing prostate carcinoma is a method using a total amount of PSA (that is, a total amount of free PSA and bound PSA, hereinafter abbreviated as "total PSA value") in serum as an index. The reference value (normal value) of the total PSA value is less than 4 ng/mL. In a case where the prostate is affected by prostate carcinoma, the total PSA value in serum rises.

However, it is known that the total PSA value often becomes higher even in a case where there is a prostate disease other than prostate carcinoma, such as benign prostatic hyperplasia or prostatitis. In addition, the range where the total PSA value is 4.1 to 10 ng/mL is called a so-called gray zone. In a case of a patient whose total PSA value is in the gray zone, it is difficult to distinguish between prostate carcinoma and prostatic hypertrophy by the total PSA value.

Therefore, for patients with high values and patients in the gray zone as a result of the test using the total PSA value as an index, it is necessary to distinguish whether the patients are suffering from prostate carcinoma or are suffering from other diseases such as benign prostatic hyperplasia. Usually, ultrasonic examination and further tissue examination (biopsy) are carried out for such a purpose. However, there were many cases in which the biopsy results determined that the disease was not prostate carcinoma. That is, there were many cases where an excessive amount of tests was carried out, which resulted in biopsies on patients who would otherwise not have to undergo biopsies. Moreover, the biopsy involves a risk of infection and presents a high physical and financial burden on the patients.

Therefore, additional diagnoses using various indexes have been attempted for the purpose of improving a diagnostic specificity and avoiding an unnecessary biopsy.

For example, in a diagnosis using a ratio of free PSA/total PSA as an index, the ratio of free PSA/total PSA tends to be lower in prostate carcinoma and higher in benign prostatic hyperplasia (Non-Patent Literature 3).

In addition, the volume of the prostate increases in a case where the prostate has any disease (prostate carcinoma, prostatic hyperplasia, or the like). Therefore, a diagnosis using an index that combines the volume of the prostate and the value related to PSA, for example, a diagnosis using a PSA density (PSAD, PSA amount/prostate volume) as an index, is carried out as one option for determining prostate carcinoma. It is said that the higher the PSA density, the more likely it is to have prostate carcinoma.

Furthermore, since PCA3 (prostate cancer antigen 3) is expressed at a high level in a prostate carcinoma patient, a PCA3 test method for detecting PCA3 RNA in urine collected from the patient is known. The PCA3 test is commonly carried out in routine clinical practice to monitor the likelihood of having a disease of the prostate in patients with abnormal total PSA test results and negative biopsy results.

In addition, prostate carcinoma is also diagnosed using an index called Prostate Health Index (PHI), which is a combination of total PSA, free PSA, and p2PSA (PSA precursor). PHI is calculated by [p2PSA/free PSA $\times$ PSA0.5]. In a case where PHI is 25 or higher, it is determined that a biopsy is necessary even in a case where the total PSA value is below the reference value.

In addition, it was revealed that PSA in which a terminal sialic acid residue of a glycan is linked to galactose through an $\alpha$(2,3) linkage, rather than PSA in which the terminal sialic acid residue of the glycan is linked to galactose through an $\alpha$(2,6) linkage, increases in the sera of prostate carcinoma patients (Non-Patent Literature 2).

Non-Patent Literature 4 describes an automated micro-total immunoassay system for measuring cancer-associated $\alpha$2,3-linked sialyl N-glycan-carrying prostate-specific antigen.

Moreover, Non-Patent Literature 5 relates to the diagnostic accuracy of the age-adjusted and prostate volume-adjusted biopsy method in males with prostate specific antigen levels of 4.1-10.0 ng/mL.

Patent Literature 2 relates to a method and a kit for distinguishing between prostate carcinoma and benign prostatic hyperplasia.

Ohyama et al. have found that the ratio of the amount of free PSA having a glycan in which the terminal sialic acid residue of the glycan is α(2,3)-linked to a second galactose residue from the terminal of the glycan to the amount of free PSA in a sample is an index for determining whether or not prostate carcinoma is developed. Then, Ohyama et al. have found that it can be determined that prostate carcinoma is developed or the probability of developing prostate carcinoma is high in a case where the ratio is 40% or higher and it can be determined that the malignancy of prostate carcinoma is high in a case where the ratio is 47% or higher. A patent application has been filed based on these findings (Patent Literature 1).

Citation List

Patent Literature

Patent Literature 1: WO 2017/130578A
Patent Literature 2: EP 2 908 136 A1

Non-Patent Literature

Non-Patent Literature 1: Stamey T. A. et al., N. Engl. J. Med., 1987, vol. 317, pp. 909 to 916
Non-Patent Literature 2: Tajiri M., Ohyama C., Wada Y, Glycobiolgy, 2008, vol. 18, pp. 2 to 8
Non-Patent Literature 3: Suzuki H. et al., Urology, 2006, vol. 67, No. 1, pp. 131 to 136.
Non-Patent Literature 4: Tomokazu Ishikawa, et al., International Journal of Molecular Sciences, February 2017, vol. 18, no. 2, p. 470.
Non-Patent Literature 5: Kazuto Ito, et al., Cancer, American Cancer Society, 31 October, 2022, vol. 95, no. 10, pp. 2112 to 2119.

**Summary of Invention**

Technical Problem

However, it is difficult to improve a probability of avoiding an unnecessary biopsy (biopsy avoidance rate) of a patient by any of the determination methods as described above. Therefore, the current situation is that the patient still has to undergo an excessive amount of tests in order to obtain a definitive diagnosis of prostate carcinoma.

The present invention has been made in view of the above situation, and an object thereof is to provide a novel determination method which is capable of determining prostate carcinoma with a higher biopsy avoidance rate.

Solution to Problem

Hereinafter, "prostate specific antigen" is abbreviated as "PSA".

As a result of extensive research to solve the problems, the present inventors have found that a ratio between "a volume of the prostate of a subject" and "a ratio between an amount of free PSA and an amount of free PSA having a glycan in which a terminal sialic acid residue of the glycan is α(2,3)-linked to a second galactose residue from a terminal of the glycan in a sample derived from the same subject" is an index for determining whether or not prostate carcinoma is developed. As a result of further extensive research, the present inventors have found that the biopsy avoidance rate is improved by carrying out prostate carcinoma determination using the ratio as an index. The present invention has been completed based on these findings.

That is, the present invention provides a method for determining prostate carcinoma, a method for obtaining data for carrying out a prostate carcinoma determination and use of a kit for determining prostate carcinoma as defined in the claims.

The method for determining prostate carcinoma according to the present invention can be used as a method for assisting a diagnosis by a doctor or the like.

Advantageous Effects of Invention

The method for determining prostate carcinoma according to the present invention can determine prostate carcinoma with a high biopsy avoidance rate, and therefore the number of subjects requiring a biopsy can be reduced. In other words, unnecessary biopsies for patients without prostate carcinoma can be avoided with high probability.

**(Brief Description of Drawings)**

Fig. 1 is a schematic diagram of an example of a glycan in which a terminal sialic acid residue of the glycan is α(2,3)-linked to a second galactose residue from a terminal of the glycan.
Fig. 2 is a diagram showing a method for preparing a DNA-labeled anti-PSA antibody in Example 1.
Fig. 3 is a schematic diagram of a microchip used in Example 1.
Fig. 4 is a schematic diagram of an in-chip flow channel of the microchip used in Example 1.
Fig. 5 shows the results of an ROC analysis obtained on the basis of the measurement results obtained in Example 1.

**Description of Embodiments**

<<Regarding PSA according to present invention>>

The "bound PSA" according to the present invention is PSA commonly referred to as "bound PSA". That is, the "bound PSA" according to the present invention refers to "PSA that forms a complex by binding to a binding protein such as α1-antichymotrypsin or α2-macroglobulin".

The "free PSA" according to the present invention is PSA commonly referred to as "free PSA". The "free PSA" according to the present invention refers to "PSA not bound to a binding protein such as α1-antichymotrypsin or α2-macroglobulin".

The "glycan in which a terminal sialic acid residue of the glycan is α(2,3)-linked to a second galactose residue from the terminal of the glycan" according to the present invention refers to a glycan in which the terminal (non-reducing terminal) of the glycan has a structure of Sia α2→3 Gal (hereinafter, sometimes abbreviated as "α(2,3) glycan"). The terminal sialic acid residue may be, for example, N-acetylneuraminic acid.

The "α(2,3) glycan" according to the present invention specifically refers to the following structure.

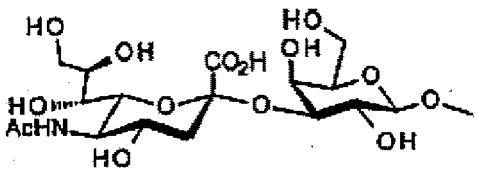

An example of the structure of the glycan of PSA having an "α(2,3) glycan" according to the present invention is shown in Formula (I).

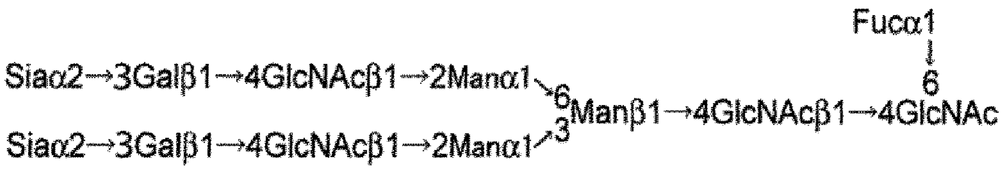
(I)

An example of PSA having the glycan of Formula (I) is shown in a schematic diagram in Fig. 1. In Fig. 1, the terminal sialic acid residue of the glycan is α(2,3)-linked to the second galactose residue from the terminal of the glycan. In addition, in Fig. 1, the glycan is linked to the asparagine residue (N) of the amino acid sequence of isoleucine-arginine-asparagine-lysine (IRNK) of the PSA protein.

The PSA having an α(2,3) glycan according to the present invention appears in the serum of patients with prostate carcinoma (Non-Patent Literature 2).

The "free PSA having an α(2,3) glycan" according to the present invention refers to a PSA which is a free PSA and has an α(2,3) glycan. Hereinafter, it is abbreviated as "α(2,3) free PSA".

«Method for determining prostate carcinoma»

The method for determining prostate carcinoma according to the present invention is "a method comprising measuring an amount of a free prostate specific antigen and an amount of a free PSA having an α(2,3) glycan, the free PSA having an α(2,3) glycan being a free prostate specific antigen having a glycan in which a terminal sialic acid residue of the glycan is α(2,3)-linked to a second galactose residue from a terminal of the glycan, in a sample derived from a subject;

obtaining a ratio 1 between the amount of the free prostate specific antigen and the amount of the free PSA having an α(2,3) glycan,
obtaining a ratio 2 between the ratio 1 and a volume of the prostate of the subject; and
determining prostate carcinoma based on the obtained ratio 2".

The method for determining prostate carcinoma according to the present invention can be used as a method for assisting a diagnosis by a doctor or the like.

<1. Sample derived from subject>

The sample derived from a subject according to the present invention (hereinafter, also referred to as "test sample" or simply "sample") is a sample derived from a human who is a subject, and examples thereof include blood, plasma, serum, semen, bladder wash, urine, tissue extract, prostate tissue section, prostate tissue biopsy sample, and those prepared therefrom. Of these, serum and plasma are preferable. Serum is particularly preferable.

<2. Method for obtaining ratio 1>

The ratio 1 according to the present invention is a "ratio between an amount of free PSA and an amount of α(2,3) free PSA" in a sample derived from a subject.
Hereinafter, a method for measuring the "amount of free PSA" and a method for measuring the "amount of α(2,3) free PSA" according to the ratio 1 will be described.

(1) Method for measuring amount of free PSA

The amount of free PSA according to the present invention is a total amount of free PSA in the sample. The amount of free PSA can also be calculated as a sum of the amount of α(2,3) free PSA and the amount of free PSA other than α(2,3) free PSA.
The free PSA other than the α(2,3) free PSA according to the present invention refers to free PSA having no α(2,3) glycan.
The method for measuring an amount of free PSA in a sample according to the present invention may be, for example,

(1)-1. a method for directly measuring an amount of free PSA in a sample, or
(1)-2. a method for measuring amount of α(2,3) free PSA and amount of free PSA other than α(2,3) free PSA in sample, and obtaining amount of free PSA from sum thereof.

(1)-1. Method for directly measuring amount of free PSA in sample

The method for directly measuring an amount of free PSA may be, for example, a known method for measuring an amount of free PSA. For example, a known immunoassay using an antibody that specifically binds to free PSA can be used.
An anti-PSA antibody according to the present invention is an antibody having an affinity for PSA. The anti-PSA antibody is specifically an antibody having an affinity for a core protein of PSA. That is, the anti-PSA antibody according to the present invention includes an antibody that is capable of binding to free PSA, and bound PSA and an antibody that specifically binds to free PSA (anti-free PSA antibody).
In a case where the anti-PSA antibody is an antibody that specifically binds to free PSA, it is designated as "anti-free PSA antibody". In addition, in a case where the anti-PSA antibody includes an antibody capable of binding to free PSA and bound PSA and an anti-free PSA antibody, it is simply described as "anti-PSA antibody".
It should be noted that, in the present specification, the phrase "having an affinity" means, for example, "binding to".
The anti-PSA antibody according to the present invention may be any anti-PSA antibody having the properties

as described above and may be a commercially available anti-PSA antibody or an anti-PSA antibody appropriately prepared by a conventional method, each of which may be a monoclonal antibody or a polyclonal antibody. In addition, these antibodies may be used alone or in an appropriate combination thereof.

In a case where the anti-PSA antibody according to the present invention is a monoclonal antibody, an origin thereof is not particularly limited, and any of a commercially available monoclonal anti-PSA antibody or a monoclonal anti-PSA antibody produced by a method known per se utilizing a cell fusion technique, a gene recombination technique, or the like [Eur. J. Immunol., 6, 511 (1976)] and having the properties as described above can be used.

In a case where the anti-PSA antibody according to the present invention is a polyclonal antibody, an origin thereof is not particularly limited, and examples of the polyclonal anti-PSA antibody include those derived from rabbits, rats, mice, sheep, goats, horses, and the like and having the properties as described above. A commercially available polyclonal anti-PSA antibody product, or a polyclonal anti-PSA antibody obtained by the method described in, for example, "Introduction to Immunology Experiments, 2nd edition, Choku Matsuhashi et al., Academic Publishing Center, 1981" may be used.

In addition, the anti-PSA antibody according to the present invention may be Fab, Fab', $F(ab')_2$, Fv, Fd, single chain Fv (scFv), disulfide linked Fv (sdFv), $V_L$, $V_H$, diabody ($(V_L-V_H)_2$ or $(V_H-V_L)_2$), triabody (trivalent antibody), tetrabody (tetravalent antibody), minibody ($(scFv-C_H3)_2$), IgG-delta-CH2, scFv-Fc, $(scFv)_2$-Fc fragment, or the like of an antibody.

Among the anti-PSA antibodies according to the present invention, examples of commercially available products of the antibody capable of binding to free PSA and bound PSA include Anti PSA monoclonal antibody PSA10 (Anti PSA monoclonal antibody clone No. PSA10, FUJIFILM Wako Pure Chemical Corporation), Anti PSA monoclonal antibody (5A6) (HyTest Ltd.), Anti PSA monoclonal antibody (5G6) (HyTest Ltd.), Anti PSA monoclonal antibody (PS6) (HyTest Ltd.), Anti PSA monoclonal antibody (PSA14) (FUJIFILM Wako Pure Chemical Corporation), Anti-Prostate Specific Antigen antibody (EP1588Y) (Abcam Plc.), Anti-Prostate Specific Antigen antibody (A67-B/E3) (Abcam Plc.), Anti-Prostate Specific Antigen antibody (35H9) (Abcam Plc.), Anti-Prostate Specific Antigen antibody (KLK3/801) (Abcam Plc.), Anti-Prostate Specific Antigen antibody (3E6) (Abcam Plc.), Anti-Prostate Specific Antigen antibody (8301) (Abcam Plc.), Anti-Prostate Specific Antigen antibody (A5D5) (Abcam Plc.), Anti-Prostate Specific Antigen antibody (PSA 28/A4) (Abcam Plc.), and Anti-Prostate Specific Antigen antibody (1H12) (Abcam Plc.).

Among the anti-PSA antibodies according to the present invention, examples of commercially available products of the anti-free PSA antibody include Anti PSA monoclonal antibody PSA12 (Anti PSA monoclonal antibody clone No. PSA12, FUJIFILM Wako Pure Chemical Corporation), Anti PSA monoclonal antibody (8A6) (HyTest Ltd.), Anti PSA monoclonal antibody (PS 1) (HyTest Ltd.), Anti PSA monoclonal antibody (clone 108) (Anogen-Yes Biotech Laboratories Ltd.), Anti-Prostate Specific Antigen antibody (PS2) (Abcam Plc.), and Anti-Prostate Specific Antigen antibody (2H9) (Abcam Plc.).

The anti-PSA antibody according to the present invention may be labeled with a detectable labeling substance.

Examples of the labeling substance used for labeling the antibody include all the labeling substances commonly used in the related art, including enzymes such as alkaline phosphatase, β-galactosidase, peroxidase, microperoxidase, glucose oxidase, glucose-6-phosphate dehydrogenase, acetylcholinesterase, malate dehydrogenase, and luciferase; radioactive isotopes such as $^{99m}Tc$, $^{131}I$, $^{125}I$, $^{14}C$, $^{3}H$, $^{32}P$, and $^{35}S$; fluorescent substances such as HiLyte 647 (manufactured by AhaSpec Inc.), fluorescein, dansyl, fluorescamine, coumarin, naphthylamine, fluorescein isothiocyanate (FITC), rhodamine, rhodamine X isothiocyanate, sulforhodamine 101, lucifer yellow, acridine, acridine isothiocyanate, riboflavin, and derivatives thereof; luminescent substances such as luciferin, isoluminol, luminol, and a bis(2,4,6-trifluorophenyl)oxalate; a substance having absorption in an ultraviolet region such as phenol, naphthol, anthracene, and derivatives thereof; substances having properties as spin labeling agents represented by compounds having an oxyl group such as 4-amino-2,2,6,6-tetramethylpiperidine-1-oxyl, 3-amino-2,2,5,5-tetramethylpyrrolidine-1-oxyl, and 2,6-di-t-butyl-α-(3,5-di-t-butyl-4-oxo-2,5-cyclohexadien-1-ylidene)-p-tolyloxyl; HiLyte coloring agents such as HiLyte Fluor 647, HiLyte Fluor 488, HiLyte Fluor 555, HiLyte Fluor 680, and HiLyte Fluor 750 (all of which are trade names of HiLyte Bioscience, Inc.); Alexa coloring agents such as Alexa Fluor Dye 350, Alexa Fluor Dye 430, Alexa Fluor Dye 488, Alexa Fluor Dye 532, Alexa Fluor Dye 546, Alexa Fluor Dye 555, Alexa Fluor Dye 568, Alexa Fluor Dye 594, Alexa Fluor Dye 633, Alexa Fluor Dye 647, Alexa Fluor Dye 660, Alexa Fluor Dye 680, Alexa Fluor Dye 700, and Alexa Fluor Dye 750 (all of which are trade names of Molecular Probes, Inc.); CyDye coloring agents such as Cy3, Cy3.5, Cy5, Cy5.5, and Cy7 (all of which are trade names of Amersham Biosciences, Inc.); and coloring agents such as Coomassie Brilliant Blue R250 and Methyl Orange.

Examples of the method for binding the labeling substance to an anti-PSA antibody include labeling methods known per se which are used commonly in EIA, RIA, or FIA known per se (for example, the method described in "Medical Chemistry Experimental Course", vol. 8, edited by Yuichi Yamamura, the 1st edition, Nakayama Shoten Co., Ltd., 1971; "Illustrative Fluorescent Antibodies", Akira Kawaoi, the 1st edition, Soft Science Inc., 1983; "Enzyme Immunoassays", Eiji Ishikawa, Tadashi Kawai, Kiyoshi Muroi ed., the 2nd edition, Igaku-Shoin Ltd., 1982; and the like). A method for binding a labeling substance to an antibody by a conventional method utilizing a reaction of avidin (or streptavidin) with biotin can also be used. These labeling methods may be appropriately carried out.

Specific examples of the method for directly measuring an amount of free PSA in a sample according to the present invention include the following methods.

[Method i]

A sample is reacted with an anti-free PSA antibody labeled with a detectable labeling substance to produce a complex of the labeled anti-free PSA antibody and free PSA. Then, an amount of the complex is measured by measuring the signal derived from the labeling substance of the labeled anti-free PSA antibody that constitutes the complex. Based on the obtained measurement value, an amount of free PSA in the sample is obtained by a conventional method. Before measuring the amount of the complex, an operation of removing the labeled anti-free PSA antibody that has not bound to free PSA may be appropriately carried out.

[Method ii]

A sample is reacted with a first antibody that is an anti-PSA antibody, and a labeled second antibody labeled with a labeling substance capable of detecting the anti-PSA antibody to produce a complex of the first antibody, free PSA, and the labeled second antibody. Then, an amount of the complex is measured by measuring the signal derived from the labeling substance of the labeled second antibody that constitutes the complex. Based on the obtained measurement value, an amount of free PSA in the sample is obtained by a conventional method. Before measuring the amount of the complex, an operation of removing the first antibody and the labeled second antibody that have not bound to free PSA may be appropriately carried out.

In the [Method ii], at least one of the first antibody or the second antibody is an anti-free PSA antibody. In addition, the epitopes of the first antibody and the second antibody are preferably different.

In the [Method i] and [Method ii], the amount of free PSA may be obtained by conversion into a quantitative value by a conventional method using the results obtained by carrying out the measurement in the same manner using a free PSA standard of known concentration in advance. That is, the signal is measured by using free PSA of known concentration and using the same reagent as the one used to measure the amount of free PSA in the sample, and carrying out the same operation. A calibration curve of the obtained measurement value and the concentration of the free PSA standard used is prepared. The amount of free PSA in the sample is obtained by fitting the signal measurement value obtained by the measurement using the sample to the calibration curve.

The first antibody used in the [Method ii] is preferably immobilized on a solid phase. The first antibody immobilized on the solid phase is preferably an anti-free PSA antibody.

In a case where an antibody immobilized on a solid phase is used, the labeled secondary antibody that has not bound to free PSA can be separated by a known B/F separation method.

Examples of the solid phase include insoluble carriers used in common immunoassay and the like. Specific examples of the insoluble carrier include synthetic polymer compounds such as polystyrene, polypropylene, polyacrylic acid, polymethacrylic acid, polyacrylamide, polyglycidyl methacrylate, polyvinyl chloride, polyethylene, polychlorocarbonate, silicone resin, and silicone rubber, and inorganic substances such as porous glass, ground glass, ceramics, alumina, silica gel, activated charcoal, and metal oxide. In addition, these insoluble carriers can be used in a wide variety of forms such as a microtiter plate, a bead, a tube, a dedicated tray in which a large number of tubes are integrally molded, a disk-like piece, and a fine particle (latex particle).

The method for immobilizing an anti-PSA antibody on the insoluble carrier is not particularly limited as long as it is carried out by bringing the anti-PSA antibody into contact with the insoluble carrier. A supporting method (for example, a so-called physical adsorption method) well known per se and commonly used in the related art is mentioned as a typical method.

In addition, in a case where a commercially available insoluble carrier is used, the anti-PSA antibody may be immobilized on the insoluble carrier according to the immobilization method recommended in the instruction manual.

The method for measuring the signal derived from the labeling substance of the labeled anti-PSA antibody described above varies depending on the type of the labeling substance, but the measurement of the signal may be carried out according to a predetermined method depending on the property possessed by the labeling substance that can be detected by any method. For example, in a case where the labeling substance is an enzyme, the measurement may be carried out according to a common procedure of immunoassay, for example, the method described in "Enzyme Immunoassays" (Proteins, Nucleic Acids, and Enzymes, separate volume No. 31, edited by Tsunehiro Kitagawa, Toshio Nanbara, Akio Tsuji, and Eiji Ishikawa, pp. 51 to 63, Kyoritsu Shuppan Co., Ltd., 1987), and in a case where the labeling substance is a radioactive substance, the measurement may be carried out, for example, according to a common procedure which is currently carried out in RIA, and using by appropriately selecting and using a measurement instrument such as an immersion type GM counter, a liquid scintillation counter, a well type scintillation counter, or a counter for HPLC, depending on the type and intensity of radiation generated by the radioactive substance (for example, refer to

"Medical Chemistry Experimental Course", vol. 8, edited by Yuichi Yamamura, the 1st edition, Nakayama Shoten Co., Ltd., 1971). In addition, in a case where the labeling substance is a fluorescent substance, the measurement may be carried out according to a common procedure which is carried out in FIA using a measurement instrument such as a spectrofluorometer, for example, according to the method described in "Illustrative Fluorescent Antibodies", Akira Kawaoi, the 1st edition, Soft Science Inc., 1983; and in a case where the labeling substance is a luminescent substance, the measurement may be carried out according to a common procedure using a measurement instrument such as a photo counter, for example, according to the method described in "Enzyme Immunoassays" (Proteins, Nucleic Acids, and Enzymes, separate volume No. 31, edited by Tsunehiro Kitagawa, Toshio Nanbara, Akio Tsuji, and Eiji Ishikawa, pp. 252 to 263, Kyoritsu Shuppan Co., Ltd., 1987). Furthermore, in a case where the labeling substance is a substance which has absorption in an ultraviolet region, the measurement may be carried out by a common procedure using a measurement instrument such as a spectrophotometer; and in a case where the labeling substance has a spin property, the measurement may be carried out by a common procedure using electron spin resonance equipment, for example, according to the method described in "Enzyme Immunoassays" (Proteins, Nucleic Acids, and Enzymes, separate volume No. 31, edited by Tsunehiro Kitagawa, Toshio Nanbara, Akio Tsuji, and Eiji Ishikawa, pp. 264 to 271, Kyoritsu Shuppan Co., Ltd., 1987).

The amount of free PSA may be measured using a commercially available kit for measuring free PSA. Examples of such a kit include Human Circulating Cancer BioMarker Panel 1 Select Kit (manufactured by Luminex Corporation), Free PSA·Abbott (manufactured by Abbott Laboratories, Inc.), Lumipulse Free PSA (manufactured by Fujirebio, Inc.), Vitros free PSA (manufactured by Ortho Clinical Diagnostics, Inc.), ST AIA-PACK free PSA (manufactured by Tosoh Corporation), and ECLusys™ reagent free PSA (manufactured by Roche Diagnostics GmbH).

In the method of (1)-1, all of the separation/measurement devices and various reagents that are commonly used in the art of immunoassays and the like known per se can be used. Concentrations of anti-PSA antibodies and reagents used for the measurement may be appropriately selected from the concentration ranges commonly used in the related art. The labeling substance used for labeling the anti-PSA antibody according to the present invention is appropriately set according to the measurement method, the measurement device, and the like for measuring the labeling substance. In addition, the type of the solid phase is appropriately selected according to the measurement device to be used and the measurement method to be carried out. The measurement conditions and the like (such as reaction temperature, reaction time, pH at the time of reaction, measurement wavelength, and measurement device) in a case of carrying out the measurement may be appropriately selected according to a method known per se.

The method of (1)-1 is not limited to a manual method, and can be sufficiently used for a measurement system using an automatic analyzer, by which the measurement can be carried out easily and quickly. There are no particular restrictions on a combination of reagents or the like in a case where the measurement is carried out using the manual method or the automatic analyzer. A combination of reagents or the like which is considered to be the best may be selected and used appropriately, depending on the environment and model of the applied automatic analyzer, or in consideration of other factors.

In the method of (1)-1, the [Method ii], in which an anti-free PSA antibody bound to an insoluble carrier is used as the first antibody is more preferable.

(1)-2. Method for measuring amount of α(2,3) free PSA and amount of free PSA other than α(2,3) free PSA in sample, and obtaining amount of free PSA from sum of amounts thereof.

Examples of the method include a method using a substance having an affinity for an α(2,3) glycan (hereinafter, abbreviated as "affinity substance with α(2,3) glycan affinity"), and mass spectrometry, among which a method using an affinity substance with α(2,3) glycan affinity is preferable.

Hereinafter, the "method for measuring an amount of α(2,3) free PSA and an amount of free PSA other than α(2,3) free PSA in a sample, using an affinity substance with α(2,3) glycan affinity and obtaining an amount of free PSA from a sum of amounts thereof' will be described. The details of the mass spectrometry are described at the end of the section "(3) Method for measuring ratio 1" which will be described later.

[Affinity substance with α(2,3) glycan affinity]

The affinity substance with α(2,3) glycan affinity according to the present invention used in the method is a substance having an affinity for an α(2,3) glycan. Preferred is a substance that has an affinity for an α(2,3) glycan, but has no affinity for other glycans (for example, an α(2,6) glycan which is a glycan in which the terminal sialic acid residue of the glycan is α(2,6)-linked to the second galactose residue from the terminal of the glycan) and is specific for an α(2,3) glycan.

The affinity substance with α(2,3) glycan affinity according to the present invention may be, for example, a lectin or antibody having such properties. Of these, a lectin is preferable. Hereinafter, a lectin and an antibody as the

affinity substance with α(2,3) glycan affinity will be described in more detail.

· Affinity substance with α(2,3) glycan affinity: Lectin

Examples of the lectin used as the affinity substance with α(2,3) glycan affinity according to the present invention include lectins having an affinity for an α(2,3) glycan. A lectin specific for an α(2,3) glycan, which has an affinity for an α(2,3) glycan but no affinity for other glycans, is preferable.

Examples of the lectin having such properties include plant lectins such as MAA which is a lectin derived from *Maackia amurensis,* and ACG which is a lectin derived from *Agrocybe cylindracea,* and animal lectins such as CD169 (sialic acid-binding Ig-like lectin 1), rat MAG, CD328, and siglec-9 (sialic acid-binding Ig-like lectin-9). Among them, MAA is preferable.

The lectin may be labeled with a detectable labeling substance.

Examples of the labeling substance used for labeling the lectin include a fluorescent coloring agent (fluorescein isothiocyanate (FITC), Cy5, Alexa Fluor 647, or the like), an enzyme (horseradish-derived peroxidase), a coenzyme, a chemiluminescent substance, a radioactive substance ($^{32}P$, $^{14}C$, $^{125}I$, $^{3}H$, $^{131}I$, or the like), and a labeling substance such as biotin. In addition, the labeling substance may be bound directly to a lectin, or may be bound to a lectin through a suitable spacer. The lectin may be labeled by a per se known labeling method corresponding to the type of the labeling substance.

· Affinity substance with α(2,3) glycan affinity: Antibody

Examples of the antibody used as the affinity substance with α(2,3) glycan affinity according to the present invention include antibodies having an affinity for an α(2,3) glycan. An antibody having a specific affinity for an α(2,3) glycan is preferable. An antibody that has an affinity for an α(2,3) glycan but no affinity for other glycans (for example, an α(2,6) glycan) is particularly preferable.

The type of the antibody used as the affinity substance with α(2,3) glycan affinity according to the present invention is not particularly limited as long as it has the properties as the affinity substance with α(2,3) glycan affinity described above, and may be a commercially available product or a product appropriately prepared by a conventional method. In addition, the antibody may be a monoclonal antibody or a polyclonal antibody. In addition, these antibodies may be used alone or in an appropriate combination thereof.

In a case where the antibody used as the affinity substance with α(2,3) glycan affinity is a monoclonal antibody, an origin thereof is not particularly limited. Any of a commercially available monoclonal antibody or a monoclonal antibody produced by a method known per se using a cell fusion technique, a gene recombination technique, or the like [Eur. J. Immunol., 6, 511 (1976)] and having the properties as the affinity substance with α(2,3) glycan affinity can be used.

In a case where the antibody used as the affinity substance with α(2,3) glycan affinity is a polyclonal antibody, an origin thereof is not particularly limited, and examples of such a polyclonal antibody include those derived from rabbits, rats, mice, sheep, goats, horses, and the like and having the properties as the affinity substance with α(2,3) glycan affinity. A commercially available polyclonal antibody product, or a polyclonal antibody obtained by the method described in, for example, "Introduction to Immunology Experiments, 2nd edition, Choku Matsuhashi et al., Academic Publishing Center, 1981" may be used.

The antibody used as the affinity substance with α(2,3) glycan affinity may be Fab, Fab', $F(ab')_2$, Fv, Fd, single chain Fv (scFv), disulfide linked Fv (sdFv), $V_L$, $V_H$, diabody ($(V_L-V_H)_2$ or $(V_H-V_L)_2$), triabody (trivalent antibody), tetrabody (tetravalent antibody), minibody ($(scFv-C_H3)_2$), IgG-delta-CH2, scFv-Fc, $(scFv)_2$-Fc fragment, or the like of an antibody.

Examples of commercially available products of the antibody used as the affinity substance with α(2,3) glycan affinity according to the present invention include Anti Siaα2-3, Monoclonal Antibody (HYB4) (manufactured by FUJIFILM Wako Pure Chemical Corporation), Anti GM3(Neu Ac), Monoclonal Antibody (Clone: M2590) (manufactured by FUJIFILM Wako Pure Chemical Corporation), Anti-Sialyl Lea Antigen, Monoclonal Antibody (MSW113) (manufactured by FUJIFILM Wako Pure Chemical Corporation), Anti-GM3 Monoclonal Antibody (manufactured by Tokyo Chemical Industry Co., Ltd.), Anti-Sialyl Lewis A Monoclonal Antibody (1H4) (manufactured by Tokyo Chemical Industry Co., Ltd.), and Anti-Sialyl Lewis A Monoclonal Antibody (NKH3) (manufactured by GlycoNex Inc.).

The antibody used as the affinity substance with α(2,3) glycan affinity according to the present invention may be labeled with a detectable labeling substance.

Examples of the labeling substance used for labeling the antibody used as the affinity substance with α(2,3) glycan affinity according to the present invention and the binding method between the labeling substance and the antibody include the same ones as described for the labeling substance used for labeling the anti-PSA antibody and the method of binding the labeling substance to the antibody, in the section "(1)-1 Method for directly measuring amount of free PSA in sample".

The "(1)-2. Method for measuring amount of α(2,3) free PSA and amount of free PSA other than α(2,3) free

PSA in sample, using affinity substance with α(2,3) glycan affinity and obtaining amount of free PSA from sum thereof' may be, for example,

(1)-2-1. a method for separately measuring an amount of α(2,3) free PSA and an amount of free PSA other than α(2,3) free PSA in a sample, using an affinity substance with α(2,3) glycan affinity and obtaining an amount of free PSA from a sum thereof, or
(1)-2-2. a method for separately measuring both an amount of α(2,3) free PSA and an amount of free PSA other than α(2,3) free PSA in a sample, in one step, using an affinity substance with α(2,3) glycan affinity and obtaining an amount of free PSA from a sum thereof.

(1)-2-1. Method for separately measuring amount of α(2,3) free PSA and amount of free PSA other than α(2,3) free PSA in sample, using affinity substance with α(2,3) glycan affinity and obtaining amount of free PSA from sum thereof

Examples of the method include the following methods.
"A sample is reacted with an affinity substance with α(2,3) glycan affinity to form a complex of α(2,3) free PSA and the affinity substance with α(2,3) glycan affinity. Next, the complex is separated from a solution containing free PSA other than α(2,3) free PSA that did not bind to the affinity substance with α(2,3) glycan affinity. The amount of α(2,3) free PSA is obtained by measuring the amount of free PSA in the separated complex. In addition, the amount of free PSA other than α(2,3) free PSA is obtained by measuring the amount of free PSA in the separated solution. The amount of free PSA is obtained by calculating a sum of the obtained amount of α(2,3) free PSA and the obtained amount of free PSA other than α(2,3) free PSA.".
Examples of the affinity substance with α(2,3) glycan affinity used in the method of (1)-2-1 include the affinity substances with α(2,3) glycan affinity according to the present invention. The lectin used as the affinity substance with α(2,3) glycan affinity or the antibody used as the affinity substance with α(2,3) glycan affinity is preferable. The lectin used as the affinity substance with α(2,3) glycan affinity is more preferable.
Examples of the method of (1)-2-1 include the following [Method 1] and [Method 2], among which [Method 2] is preferable.

[Method 1]

A sample is allowed to flow through a column packed with a packing material in which an affinity substance with α(2,3) glycan affinity is immobilized on a solid phase such as agarose beads. α(2,3) free PSA binds to the affinity substance with α(2,3) glycan affinity on the packing material, and free PSA other than α(2,3) free PSA is eluted from the column without binding to the affinity substance with α(2,3) glycan affinity. Therefore, the "amount of free PSA other than α(2,3) free PSA" is obtained by measuring the amount of free PSA in the eluate. Then, the column is washed with an appropriate buffer solution, and about 2 to 5-fold column volume of a lactose-containing buffer solution (0.4 M) is allowed to flow through the column to elute α(2,3) free PSA. The "amount of α(2,3) free PSA" can be obtained by measuring the amount of free PSA in the eluate.
The measurement of the amount of α(2,3) free PSA and the amount of free PSA other than α(2,3) free PSA in the [Method 1] may be carried out in the same manner as in the section "(1)-1. Method for directly measuring amount of free PSA in sample".
The "amount of free PSA" can be obtained by obtaining a sum of the obtained "amount of free PSA other than α(2,3) free PSA" and the obtained "amount of α(2,3) free PSA".

[Method 2]

A sample is brought into contact with a solid phase such as a microtiter plate or polystyrene beads on which an affinity substance with α(2,3) glycan affinity is immobilized. α(2,3) free PSA binds to the affinity substance with α(2,3) glycan affinity on the solid phase. Free PSA other than α(2,3) free PSA does not bind to the affinity substance with α(2,3) glycan affinity and is present in the liquid phase. The "amount of free PSA other than α(2,3) free PSA" can be obtained by separating the solid phase from the liquid phase and measuring the amount of free PSA in the liquid phase. Then, the amount of α(2,3) free PSA can be obtained by measuring the amount of free PSA bound to the solid phase.
The amount of PSA other than α(2,3) free PSA in the liquid phase in the [Method 2] may be measured in the same manner as in the section (1)-1.
In the [Method 2], the amount of α(2,3) free PSA bound to the solid phase may be measured, for example, by the following method.
α(2,3) free PSA bound to a solid phase is reacted with a labeled anti-free PSA antibody to form a complex of the α(2,3) free PSA and the labeled anti-free PSA antibody on the solid phase. Then, an amount of the complex is

measured by measuring the signal derived from the labeling substance of the labeled anti-free PSA antibody that constitutes the complex. The amount of α(2,3) free PSA may be obtained by conversion into quantitative values by a conventional method using the results obtained by carrying out the measurement in the same manner using an α(2,3) free PSA standard of known concentration in advance.

Here, as a method for preparing the α(2,3) free PSA standard used in the present invention, for example, the method described in Yoneyama T. et al., Biochem Biophys Res Commun. 2014 vol. 448, No. 4, pp. 390 to 396 can be mentioned. Specifically, recombinant free PSA (hereinafter, referred to as "r free PSA") is obtained according to the method disclosed in "2. Materials and methods (2.7 Forced expression of FLAG-tag-fused α(2,3) PSA)" of the literature. The obtained r free PSA includes recombinant α(2,3) free PSA (hereinafter, referred to as "r α(2,3) free PSA") and recombinant α(2,6) free PSA (hereinafter, referred to as "r α(2,6) free PSA"). The α(2,6) free PSA refers to "PSA having an α(2,6) glycan".

From the r free PSA thus obtained, r α(2,3) free PSA and r α(2,6) free PSA are separated and purified by a known method. For example, first, r α(2,3) free PSA and r α(2,6) free PSA are separated by lectin column chromatography using a lectin showing a high affinity for the sialyl α2,3-galactose structure. Next, gel filtration is carried out to purify r α(2,3) free PSA and r α(2,6) free PSA, respectively. The resulting purified r α(2,3) free PSA can be used as an "α(2,3) free PSA standard". In addition, the resulting purified r α(2,6) free PSA can be used as an "α(2,6) free PSA standard" as necessary.

The labeling substance used for labeling the anti-free PSA antibody and the method for binding the labeling substance to the antibody, used in the [Method 2], include the same ones as described for the labeling substance used for labeling the anti-PSA antibody and the method of binding the labeling substance to the antibody, in the section (1)-1. In addition, the method for measuring the signal derived from the labeling substance of the complex using the labeled anti-free PSA antibody includes the same method as that described in the section (1)-1.

Examples of the types of solid phase and the method of immobilizing the antibody to be used on the solid phase, used in the [Method 1] and [Method 2], include the same ones as described in the section (1)-1.

The "amount of free PSA" can be obtained by obtaining a sum of the obtained "amount of free PSA other than α(2,3) free PSA" and the obtained "amount of α(2,3) free PSA".

In the [Method 1] and [Method 2], all of the separation/measurement devices and various reagents that are used in the art of immunoassays and the like known per se can be used in the method. The types of the affinity substance with α(2,3) glycan affinity and the solid phase used for the measurement are appropriately selected depending on the measurement device to be used and the measurement method to be carried out. Concentrations of anti-free PSA antibodies and reagents used for the measurement may be appropriately selected from the concentration ranges commonly used in the related art. The labeling substance used for labeling the anti-free PSA antibody is appropriately selected according to the measurement method, the measurement device, and the like for measuring the labeling substance. In addition, the measurement conditions and the like (such as reaction temperature, reaction time, pH at the time of reaction, measurement wavelength, and measurement device) in a case of carrying out the measurement may be appropriately selected according to a method known per se.

(1)-2-2. Method for separately measuring both amount of α(2,3) free PSA and amount of free PSA other than α(2,3) free PSA in sample, in one step, using affinity substance with α(2,3) glycan affinity and obtaining amount of free PSA from sum thereof

Examples of the method include the following methods.

"A sample is reacted with an affinity substance with α(2,3) glycan affinity and an anti-free PSA antibody to produce a complex of the affinity substance with α(2,3) glycan affinity, α(2,3) free PSA, and the anti-free PSA antibody (first complex), and a complex of free PSA other than α(2,3) free PSA and the anti-free PSA antibody (second complex). After separating (fractionating) both complexes, the amount of the first complex and the amount of the second complex are measured whereby both α(2,3) free PSA and free PSA other than α(2,3) free PSA are separately measured in one step. The amount of free PSA is obtained by obtaining a sum of the obtained amount of the first complex and the obtained amount of the second complex."

Examples of the affinity substance with α(2,3) glycan affinity used in the method of (1)-2-2 include the affinity substances with α(2,3) glycan affinity according to the present invention. The lectin used as the affinity substance with α(2,3) glycan affinity or the antibody used as the affinity substance with α(2,3) glycan affinity is preferable. The lectin used as the affinity substance with α(2,3) glycan affinity is more preferable.

Specific examples of the method of (1)-2-2 include methods according to steps of [Method 3] to [Method 8] which will be given below.

In the methods of [Method 3] to [Method 8] which will be given below, the phrase "separating on the basis of affinity" means, for example, "separating an object to be separated on the basis of the difference in binding strength thereof. For example, "separating the first complex and the second complex on the basis of the affinity of the affinity

substance with α(2,3) glycan affinity for an α(2,3) glycan" means "separating the first complex and the second complex on the basis of the difference in the binding strength of the first complex to the affinity substance with α(2,3) glycan affinity and the binding strength of the second complex to the affinity substance with α(2,3) glycan affinity".

[Method 3]

1) a step of bring a sample, a labeled anti-free PSA antibody labeled with a labeling substance, and an affinity substance with α(2,3) glycan affinity into contact with one another to form a complex of the labeled anti-free PSA antibody, α(2,3) free PSA, and the affinity substance with α(2,3) glycan affinity (first complex), and a complex of the labeled anti-free PSA antibody and free PSA other than α(2,3) free PSA (second complex),
2) a step of separating (fractionating) the first complex and the second complex obtained in the step of 1),
3) a step of measuring the signal derived from the labeling substance of the labeled anti-free PSA antibody constituting the first complex and the second complex, thereby measuring the amount of the first complex and the amount of the second complex separated in the step of 2), and
4) a step of obtaining a sum of the amount of the first complex and the amount of the second complex obtained in the step of 3) and setting the sum as the amount of free PSA.

The first complex and the second complex may be separated on the basis of the affinity of the affinity substance with α(2,3) glycan affinity for an α(2,3) glycan or may be separated on the basis of the difference in mass, charge, size, or the like between the first complex and the second complex (for example, electrophoresis).

[Method 4]

1) a step of bring a sample, a labeled first antibody in which an anti-PSA antibody is labeled with a labeling substance, a second antibody that is an anti-PSA antibody, and an affinity substance with α(2,3) glycan affinity into contact with one another to form a complex of the labeled first antibody, α(2,3) free PSA, the second antibody, and the affinity substance with α(2,3) glycan affinity (first complex), and a complex of the labeled first antibody, free PSA other than α(2,3) free PSA, and the second antibody (second complex),
2) a step of separating (fractionating) the first complex and the second complex obtained in the step of 1),
3) a step of measuring the signal derived from the labeling substance of the labeled first antibody constituting the first complex and the second complex, thereby measuring the amount of the first complex and the amount of the second complex separated in the step of 2), and
4) a step of obtaining a sum of the amount of the first complex and the amount of the second complex obtained in the step of 3) and setting the sum as the amount of free PSA.

At least one of the first antibody or the second antibody used in the [Method 4] is an anti-free PSA antibody. The epitopes of the first antibody and the second antibody are preferably different. The first antibody is more preferably an anti-free PSA antibody.
In addition, the first complex and the second complex may be separated on the basis of the affinity of the affinity substance with α(2,3) glycan affinity for an α(2,3) glycan or may be separated on the basis of the difference in mass, charge, size, or the like between the first complex and the second complex (for example, electrophoresis).

[Method 5]

1) a step of bring a sample, a labeled first antibody in which an anti-free PSA antibody is labeled with a labeling substance, a second antibody having an affinity for an α(2,3) glycan (an affinity substance 1 with α(2,3) glycan affinity), and a lectin having an affinity for an α(2,3) glycan (an affinity substance 2 with α(2,3) glycan affinity) into contact with one another to form a complex of the labeled first antibody, α(2,3) free PSA, the second antibody, and the lectin (first complex), and a complex of the labeled first antibody and free PSA other than α(2,3) free PSA (second complex),
2) a step of separating (fractionating) the first complex and the second complex obtained in the step of 1),
3) a step of measuring the signal derived from the labeling substance of the labeled first antibody constituting the

first complex and the second complex, thereby measuring the amount of the first complex and the amount of the second complex separated in the step of 2), and
4) a step of obtaining a sum of the amount of the first complex and the amount of the second complex obtained in the step of 3) and setting the sum as the amount of free PSA.

The first complex and the second complex may be separated on the basis of the affinity of the affinity substance 1 with α(2,3) glycan affinity or the affinity substance 2 with α(2,3) glycan affinity for an α(2,3) glycan or may be separated on the basis of the difference in mass, charge, size, or the like between the first complex and the second complex (for example, electrophoresis).

[Method 6]

1) a step of bring a sample, an anti-free PSA antibody, and an affinity substance with α(2,3) glycan affinity into contact with one another to form a complex of the anti-free PSA antibody, α(2,3) free PSA, and the affinity substance with α(2,3) glycan affinity (first complex), and a complex of the anti-free PSA antibody and free PSA other than α(2,3) free PSA (second complex),
2) a step of separating (fractionating) the first complex and the second complex obtained in the step of 1),
3) a step of measuring the amount of the first complex and the amount of the second complex separated in the step of 2), and
4) a step of obtaining a sum of the amount of the first complex and the amount of the second complex obtained in the step of 3) and setting the sum as the amount of free PSA.

The first complex and the second complex may be separated on the basis of the affinity of the affinity substance with α(2,3) glycan affinity for an α(2,3) glycan or may be separated on the basis of the difference in mass, charge, size, or the like between the first complex and the second complex (for example, electrophoresis).
To measure the amount of the first complex and the amount of the second complex, for example, the amount of PSA protein of each complex may be measured. Specifically, in a case where the first complex and the second complex are separated by electrophoresis, for example, there is a method in which a known Western blotting method using the anti-PSA antibody according to the present invention as a primary antibody and using a labeled antibody labeled with a measurable labeling substance as a secondary antibody is carried out to measure the amount of PSA protein in each separated fraction.

[Method 7]

1) a step of bring a sample, a first antibody that is an anti-PSA antibody, a second antibody that is an anti-PSA antibody, and an affinity substance with α(2,3) glycan affinity into contact with one another to form a complex of the first antibody, α(2,3) free PSA, the second antibody, and the affinity substance with α(2,3) glycan affinity (first complex), and a complex of the first antibody, free PSA other than α(2,3) free PSA, and the second antibody (second complex),
2) a step of separating (fractionating) the first complex and the second complex obtained in the step of 1),
3) a step of measuring the amount of the first complex and the amount of the second complex separated in the step of 2), and
4) a step of obtaining a sum of the amount of the first complex and the amount of the second complex obtained in the step of 3) and setting the sum as the amount of free PSA.

At least one of the first antibody or the second antibody used in the [Method 7] is an anti-free PSA antibody. The epitopes of the first antibody and the second antibody are preferably different.
In addition, the first complex and the second complex may be separated on the basis of the affinity of the affinity substance with α(2,3) glycan affinity for an α(2,3) glycan or may be separated on the basis of the difference in mass, charge, size, or the like between the first complex and the second complex (for example, electrophoresis).
To measure the amount of the first complex and the amount of the second complex, for example, the amount of PSA protein of each complex may be measured. Specifically, in a case where the first complex and the second complex are separated by electrophoresis, for example, there is a method in which a known Western blotting method using the anti-PSA antibody according to the present invention as a primary antibody and using a labeled antibody

labeled with a measurable labeling substance as a secondary antibody is carried out to measure the amount of PSA protein in each separated fraction.

[Method 8]

1) a step of bring a sample, a first antibody that is an anti-free PSA antibody, a second antibody having an affinity for an α(2,3) glycan (an affinity substance 1 with α(2,3) glycan affinity), and a lectin having an affinity for an α(2,3) glycan (an affinity substance 2 with α(2,3) glycan affinity) into contact with one another to form a complex of the first antibody, α(2,3) free PSA, the second antibody, and the lectin (first complex), and a complex of the first antibody and free PSA other than α(2,3) free PSA (second complex),
2) a step of separating (fractionating) the first complex and the second complex obtained in the step of 1),
3) a step of measuring the amount of the first complex and the amount of the second complex separated in the step of 2), and
4) a step of obtaining a sum of the amount of the first complex and the amount of the second complex obtained in the step of 3) and setting the sum as the amount of free PSA.

The first complex and the second complex may be separated on the basis of the affinity of the lectin for an α(2,3) glycan or may be separated on the basis of the difference in mass, charge, size, or the like between the first complex and the second complex (for example, electrophoresis).
To measure the amount of the first complex and the amount of the second complex, for example, the amount of PSA protein of each complex may be measured. Specifically, in a case where the first complex and the second complex are separated by electrophoresis, for example, there is a method in which a known Western blotting method using the anti-PSA antibody according to the present invention as a primary antibody and using a labeled antibody labeled with a measurable labeling substance as a secondary antibody is carried out to measure the amount of PSA protein in each separated fraction.
The labeling substance used for labeling the anti-PSA antibody and the method for binding the labeling substance to the antibody, used in the [Method 1] to [Method 8], include the same ones as described for the labeling substance used for labeling the anti-PSA antibody and the method of binding the labeling substance to the antibody, in the section (1)-1. In addition, the method for measuring the signal derived from the labeling substance in the complex using the labeled anti-PSA antibody includes the same method as that described in the section (1)-1.
In the [Method 3] to [Method 8], the anti-PSA antibody and the affinity substance with α(2,3) glycan affinity may be immobilized on a solid phase depending on the measurement method.
Examples of the solid phase and the method of immobilizing each antibody to be used on the solid phase, used in the [Method 3] to [Method 8], include the same ones as described in the section (1)-1.
In the [Method 3] to [Method 8], all of the separation/measurement devices and various reagents that are used in the art of immunoassays and the like known per se can be used in the method. The types of the affinity substance with α(2,3) glycan affinity and the solid phase used for the measurement are appropriately selected depending on the device to be used and the measurement method to be carried out. Concentrations of anti-PSA antibodies and reagents used for the measurement may be appropriately selected from the concentration ranges commonly used in the related art. The labeling substance used for labeling the anti-PSA antibody is appropriately selected according to the measurement method, the measurement device, and the like for measuring the labeling substance. In addition, the measurement conditions and the like (such as reaction temperature, reaction time, pH at the time of reaction, measurement wavelength, and measurement device) in a case of carrying out the measurement may be appropriately selected according to a method known per se.
The "(1) Method for measuring amount of free PSA" is preferably the method of (1)-2 and more preferably the method of (1)-2-2. Among the methods of (1)-2-2, [Method 3], [Method 4], [Method 6], and [Method 7] are preferable, and [Method 4] is more preferable.

(2) Method for measuring amount of α(2,3) free PSA

Examples of the method include the following methods:

(2)-1. a method for directly measuring an amount of α(2,3) free PSA in a sample, and
(2)-2. a method in which a value obtained by subtracting an amount of free PSA other than α(2,3) free PSA from an amount of free PSA in a sample is taken as an amount of α(2,3) free PSA.

(2)-1. Method for directly measuring amount of α(2,3) free PSA in sample

The method may be, for example, a "method of reacting α(2,3) free PSA with an affinity substance with α(2,3) glycan affinity to produce a complex of the α(2,3) free PSA and the affinity substance with α(2,3) glycan affinity, measuring an amount of the complex, and obtaining an amount of α(2,3) free PSA based on the obtained measurement results".
Specifically, for example, according to the method described in the section (1)-2-1, α(2,3) free PSA and free PSA other than α(2,3) free PSA may be separated and the amount of α(2,3) free PSA may be measured. In this case, it is not necessary to measure the amount of free PSA other than α(2,3) free PSA.
More specifically, for example, the following [Method 1'] and [Method 2'] can be mentioned, and [Method 2'] is preferable.

[Method 1']

A sample is allowed to flow through a column packed with a packing material in which an affinity substance with α(2,3) glycan affinity is immobilized on a solid phase such as agarose beads. α(2,3) free PSA binds to the affinity substance with α(2,3) glycan affinity on the packing material, and free PSA other than α(2,3) free PSA is eluted from the column without binding to the affinity substance with α(2,3) glycan affinity. The column is washed with an appropriate buffer solution, and then a 5-fold column volume of a lactose-containing buffer solution (0.4 M) is allowed to flow through the column to elute α(2,3) free PSA. The "amount of α(2,3) free PSA" can be obtained by measuring the amount of free PSA in the eluate.
In the [Method 1'], the amount of α(2,3) free PSA may be measured in the same manner as in the section (1)-1.

[Method 2']

A sample is brought into contact with a solid phase such as a microtiter plate or polystyrene beads on which an affinity substance with α(2,3) glycan affinity is immobilized. α(2,3) free PSA binds to the affinity substance with α(2,3) glycan affinity on the solid phase. The amount of α(2,3) free PSA can be obtained by separating the solid phase and the liquid phase and measuring the amount of free PSA bound to the solid phase.
In the [Method 2'], the amount of α(2,3) free PSA bound to the solid phase may be measured, for example, by the following method.
α(2,3) free PSA bound to a solid phase is reacted with a labeled anti-free PSA antibody to form a complex of the α(2,3) free PSA and the labeled anti-free PSA antibody on the solid phase. Then, an amount of the complex is measured by measuring the signal derived from the labeling substance of the labeled anti-free PSA antibody that constitutes the complex. The amount of α(2,3) free PSA may be obtained by conversion into quantitative values by a conventional method using the results obtained by carrying out the measurement in the same manner using an α(2,3) free PSA standard of known concentration in advance.
The labeling substance used for labeling the labeled anti-free PSA antibody and the method for binding the labeling substance to the antibody, used in the [Method 2'], include the same ones as described for the labeling substance used for labeling the anti-PSA antibody and the method of binding the labeling substance to the antibody, in the section (1)-1. In addition, the method for measuring the signal derived from the labeling substance of the complex using the labeled anti-free PSA antibody includes the same method as that described in the section (1)-1.
Examples of the types of solid phase and the method of immobilizing each antibody to be used on the solid phase, used in the [Method 1'] and [Method 2'], include the same ones as described in the section (1)-1.
In the [Method 1'] and [Method 2'], all of the separation/measurement devices and various reagents that are used in the art of immunoassays and the like known per se can be used in the method. The types of the affinity substance with α(2,3) glycan affinity and the solid phase used for the measurement are appropriately selected depending on the measurement device to be used and the measurement method to be carried out. Concentrations of anti-free PSA antibodies and reagents used for the measurement may be appropriately selected from the concentration ranges commonly used in the related art. The labeling substance used for labeling the anti-free PSA antibody is appropriately selected according to the measurement method, the measurement device, and the like for measuring the labeling substance. In addition, the measurement conditions and the like (such as reaction temperature, reaction time, pH at the time of reaction, measurement wavelength, and measurement device) in a case of carrying out the measurement may be appropriately selected according to a method known per se.
In addition, the amount of α(2,3) free PSA can also be directly measured by the method of the following [Method 9] to [Method 11] using an affinity substance with α(2,3) glycan affinity labeled with a labeling substance.
Examples of the affinity substance with α(2,3) glycan affinity used in the method of the following [Method 9] to [Method 11] include the affinity substances with α(2,3) glycan affinity according to the present invention. Above all, the lectin used as the affinity substance with α(2,3) glycan affinity or the antibody used as the affinity substance with α(2,3)

glycan affinity is preferable. The lectin used as the affinity substance with α(2,3) glycan affinity is more preferable.

[Method 9]

1) a step of bring a sample, a labeled first antibody labeled with a labeling substance and having an affinity for an α(2,3) glycan (an affinity substance 1 with α(2,3) glycan affinity), a second antibody that is an anti-free PSA antibody, and a lectin having an affinity for an α(2,3) glycan (an affinity substance 2 with α(2,3) glycan affinity) into contact with one another to form a complex of the labeled first antibody, α(2,3) free PSA, the second antibody, and the lectin (first complex), and a complex of free PSA other than α(2,3) free PSA and the second antibody (second complex),
2) optionally, a step of separating the first complex and the second complex obtained in the step of 1), and
3) a step of measuring the amount of the first complex by measuring the signal derived from the labeling substance of the labeled first antibody constituting the first complex, and obtaining the amount of α(2,3) free PSA on the basis of the measurement value.

The first complex and the second complex may be separated on the basis of the affinity of the affinity substance 1 with α(2,3) glycan affinity or the affinity substance 2 with α(2,3) glycan affinity for an α(2,3) glycan or may be separated on the basis of the difference in mass, charge, size, or the like between the first complex and the second complex (for example, electrophoresis).
In the [Method 9], the amount of α(2,3) free PSA may be obtained by conversion into quantitative values by a conventional method using the results obtained by carrying out the measurement in the same manner using an α(2,3) free PSA standard of known concentration in advance.

[Method 10]

1) a step of bring a sample, a labeled affinity substance with α(2,3) glycan affinity labeled with a labeling substance, and an anti-free PSA antibody into contact with one another to form a complex of the labeled affinity substance with α(2,3) glycan affinity, α(2,3) free PSA, and the anti-free PSA antibody (first complex), and a complex of free PSA other than α(2,3) free PSA and the anti-free PSA antibody (second complex),
2) optionally, a step of separating the first complex and the second complex obtained in the step of 1), and
3) a step of measuring the amount of the first complex by measuring the signal derived from the labeling substance of the labeled affinity substance with α(2,3) glycan affinity constituting the first complex, and obtaining the amount of α(2,3) free PSA on the basis of the measurement value.

The first complex and the second complex may be separated on the basis of the affinity of the first antibody for an α(2,3) glycan or may be separated on the basis of the difference in mass, charge, size, or the like between the first complex and the second complex (for example, electrophoresis).
In the [Method 10], the amount of α(2,3) free PSA may be obtained by conversion into quantitative values by a conventional method using the results obtained by carrying out the measurement in the same manner using an α(2,3) free PSA standard of known concentration in advance.

[Method 11]

1) a step of bring a sample, a labeled affinity substance with α(2,3) glycan affinity labeled with a labeling substance, a first antibody that is an anti-PSA antibody, and a second antibody that is an anti-PSA antibody into contact with one another to form a complex of the labeled affinity substance with α(2,3) glycan affinity, α(2,3) free PSA, the first antibody, and the second antibody (first complex), and a complex of free PSA other than α(2,3) free PSA, the first antibody, and the second antibody (second complex),
2) optionally, a step of separating the first complex and the second complex obtained in the step of 1), and
3) a step of measuring the amount of the first complex by measuring the signal derived from the labeling substance of the labeled affinity substance with α(2,3) glycan affinity constituting the first complex, and obtaining the amount of α(2,3) free PSA on the basis of the measurement value.

At least one of the first antibody or the second antibody used in the [Method 11] is an anti-free PSA antibody. The epitopes of the first antibody and the second antibody are preferably different.

In addition, the first complex and the second complex may be separated on the basis of the affinity of the affinity substance with α(2,3) glycan affinity for an α(2,3) glycan or may be separated on the basis of the difference in mass, charge, size, or the like between the first complex and the second complex (for example, electrophoresis).

In the [Method 11], the amount of α(2,3) free PSA may be obtained by conversion into quantitative values by a conventional method using the results obtained by carrying out the measurement in the same manner using an α(2,3) free PSA standard of known concentration in advance.

In a case where the affinity substance with α(2,3) glycan affinity used in the [Method 9] to [Method 11] is an antibody, the labeling substance used for labeling the antibody and the method for binding the labeling substance to the antibody include the same ones as described for the labeling substance used for labeling the anti-PSA antibody and the method of binding the labeling substance to the antibody, in the section (1)-1. In addition, the method for measuring the signal derived from the labeling substance of the complex using the labeled anti-PSA antibody includes the same method as that described in the section (1)-1.

In a case where the affinity substance with α(2,3) glycan affinity used in the [Method 9] to [Method 11] is a lectin, the labeling substance used for labeling the lectin and the method for binding the labeling substance to the lectin include the same ones as described in the section of "Affinity substance with α(2,3) glycan affinity: Lectin" in the section (1)-2. In addition, as the method for measuring the signal derived from the labeling substance in the complex using the labeled lectin, a preferable method may be appropriately selected according to the method described in the section (1)-1 and depending on the type of the labeling substance to be used.

In the [Method 9] to [Method 11], the anti-PSA antibody or the affinity substance with α(2,3) glycan affinity may be immobilized on a solid phase depending on the measurement method to be carried out.

Examples of the solid phase and the method of immobilizing each antibody to be used on the solid phase, used in the [Method 9] to [Method 11], include the same ones as described in the section (1)-1. In addition, examples of the method for immobilizing an antibody that is an affinity substance with α(2,3) glycan affinity on the solid phase include the same ones as described in the section (1)-1. Immobilization of the lectin, which is an affinity substance with α(2,3) glycan affinity, on the solid phase may be carried out by an immobilization method known per se commonly used in the related art.

In the [Method 9] to [Method 11], all of the separation/measurement devices and various reagents that are used in the art of immunoassays and the like known per se can be used in the method. The types of the affinity substance with α(2,3) glycan affinity and the solid phase used for the measurement are appropriately selected depending on the measurement device to be used and the measurement method to be carried out. Concentrations of anti-PSA antibodies and reagents used for the measurement may be appropriately selected from the concentration ranges commonly used in the related art. The labeling substance used for labeling the affinity substance with α(2,3) glycan affinity is appropriately selected according to the measurement method, the measurement device, and the like for measuring the labeling substance. In addition, the measurement conditions and the like (such as reaction temperature, reaction time, pH at the time of reaction, measurement wavelength, and measurement device) in a case of carrying out the measurement may be appropriately selected according to a method known per se.

Furthermore, the amount of α(2,3) free PSA can also be measured by the method for measuring the amount of α(2,3) free PSA in the section (1)-2-2.

That is, according to the method described in the section (1)-2-2, α(2,3) free PSA and free PSA other than α(2,3) free PSA may be separated and the amount of α(2,3) free PSA may be measured. In this case, it is not necessary to measure the amount of free PSA other than α(2,3) free PSA. In addition, it is not necessary to obtain a sum of the amount of α(2,3) free PSA and the amount of free PSA other than α(2,3) free PSA.

Specifically, for example, the following methods can be mentioned.

[Method 3']

1) a step of bring a sample, a labeled anti-free PSA antibody labeled with a labeling substance, and an affinity substance with α(2,3) glycan affinity into contact with one another to form a complex of the labeled anti-free PSA antibody, α(2,3) free PSA, and the affinity substance with α(2,3) glycan affinity (first complex), and a complex of the labeled anti-free PSA antibody and free PSA other than α(2,3) free PSA (second complex),
2) a step of separating (fractionating) the first complex and the second complex obtained in the step of 1), and
3) a step of measuring the signal derived from the labeling substance of the labeled anti-free PSA antibody constituting the first complex, thereby measuring the amount of the first complex separated in the step of 2).

The first complex and the second complex may be separated on the basis of the affinity of the affinity substance with α(2,3) glycan affinity for an α(2,3) glycan or may be separated on the basis of the difference in mass, charge, size, or the like between the first complex and the second complex (for example, electrophoresis).

[Method 4']

1) a step of bring a sample, a labeled first antibody in which an anti-PSA antibody is labeled with a labeling substance, a second antibody that is an anti-PSA antibody, and an affinity substance with α(2,3) glycan affinity into contact with one another to form a complex of the labeled first antibody, α(2,3) free PSA, the second antibody, and the affinity substance with α(2,3) glycan affinity (first complex), and a complex of the labeled first antibody, free PSA other than α(2,3) free PSA, and the second antibody (second complex),
2) a step of separating (fractionating) the first complex and the second complex obtained in the step of 1), and
3) a step of measuring the signal derived from the labeling substance of the labeled first antibody constituting the first complex, thereby measuring the amount of the first complex separated in the step of 2).

At least one of the first antibody or the second antibody used in the [Method 4'] is an anti-free PSA antibody. The first antibody is more preferably an anti-free PSA antibody.
In addition, the first complex and the second complex may be separated on the basis of the affinity of the affinity substance with α(2,3) glycan affinity for an α(2,3) glycan or may be separated on the basis of the difference in mass, charge, size, or the like between the first complex and the second complex (for example, electrophoresis).

[Method 5']

1) a step of bring a sample, a labeled first antibody in which an anti-free PSA antibody is labeled with a labeling substance, a second antibody having an affinity for an α(2,3) glycan (an affinity substance 1 with α(2,3) glycan affinity), and a lectin having an affinity for a glycan in which a terminal sialic acid residue of the glycan is α(2,3)-linked to a second galactose residue from the terminal of the glycan (an affinity substance 2 with α(2,3) glycan affinity) into contact with one another to form a complex of the labeled first antibody, α(2,3) free PSA, the second antibody, and the lectin (first complex), and a complex of the labeled first antibody and free PSA other than α(2,3) free PSA (second complex),
2) a step of separating (fractionating) the first complex and the second complex obtained in the step of 1), and
3) a step of measuring the signal derived from the labeling substance of the labeled first antibody constituting the first complex, thereby measuring the amount of the first complex separated in the step of 2).

The first complex and the second complex may be separated on the basis of the affinity of the affinity substance 1 with α(2,3) glycan affinity or the affinity substance 2 with α(2,3) glycan affinity for an α(2,3) glycan or may be separated on the basis of the difference in mass, charge, size, or the like between the first complex and the second complex (for example, electrophoresis).

[Method 6']

1) a step of bring a sample, an anti-free PSA antibody, and an affinity substance with α(2,3) glycan affinity into contact with one another to form a complex of the anti-free PSA antibody, α(2,3) free PSA, and the affinity substance with α(2,3) glycan affinity (first complex), and a complex of the anti-free PSA antibody and free PSA other than α(2,3) free PSA (second complex),
2) a step of separating (fractionating) the first complex and the second complex obtained in the step of 1), and
3) a step of measuring the amount of the first complex separated in the step of 2).

The first complex and the second complex may be separated on the basis of the affinity of the affinity substance with α(2,3) glycan affinity for an α(2,3) glycan or may be separated on the basis of the difference in mass, charge, size, or the like between the first complex and the second complex (for example, electrophoresis).
To measure the amount of the first complex, for example, the amount of PSA protein of the first complex may

be measured. Specifically, in a case where the first complex and the second complex are separated by electrophoresis, for example, there is a method in which a known Western blotting method using the anti-PSA antibody according to the present invention as a primary antibody and using a labeled antibody labeled with a measurable labeling substance as a secondary antibody is carried out to measure the amount of PSA protein in the separated first complex fraction.

[Method 7']

1) a step of bring a sample, a first antibody that is an anti-PSA antibody, a second antibody that is an anti-PSA antibody, and an affinity substance with α(2,3) glycan affinity into contact with one another to form a complex of the first antibody, α(2,3) free PSA, the second antibody, and the affinity substance with α(2,3) glycan affinity (first complex), and a complex of the first antibody, free PSA other than α(2,3) free PSA and the second antibody (second complex),
2) a step of separating (fractionating) the first complex and the second complex obtained in the step of 1), and
3) a step of measuring the amount of the first complex separated in the step of 2).

At least one of the first antibody or the second antibody used in the [Method 7'] is an anti-free PSA antibody. The epitopes of the first antibody and the second antibody are preferably different.
In addition, the first complex and the second complex may be separated on the basis of the affinity of the affinity substance with α(2,3) glycan affinity for an α(2,3) glycan or may be separated on the basis of the difference in mass, charge, size, or the like between the first complex and the second complex (for example, electrophoresis).
To measure the amount of the first complex, for example, the amount of PSA protein of the first complex may be measured. Specifically, in a case where the first complex and the second complex are separated by electrophoresis, for example, there is a method in which a known Western blotting method using the anti-PSA antibody according to the present invention as a primary antibody and using a labeled antibody labeled with a measurable labeling substance as a secondary antibody is carried out to measure the amount of PSA protein in the separated first complex fraction.

[Method 8']

1) a step of bring a sample, a first antibody that is an anti-free PSA antibody, a second antibody having an affinity for an α(2,3) glycan (an affinity substance 1 with α(2,3) glycan affinity), and a lectin having an affinity for an α(2,3) glycan (an affinity substance 2 with α(2,3) glycan affinity) into contact with one another to form a complex of the first antibody, α(2,3) free PSA, the second antibody, and the lectin (first complex), and a complex of the first antibody and free PSA other than α(2,3) free PSA (second complex),
2) a step of separating (fractionating) the first complex and the second complex obtained in the step of 1), and
3) a step of measuring the amount of the first complex separated in the step of 2).

The first complex and the second complex may be separated on the basis of the affinity of the lectin for an α(2,3) glycan or may be separated on the basis of the difference in mass, charge, size, or the like between the first complex and the second complex (for example, electrophoresis).
To measure the amount of the first complex, for example, the amount of PSA protein of the first complex may be measured. Specifically, in a case where the first complex and the second complex are separated by electrophoresis, for example, there is a method in which a known Western blotting method using the anti-PSA antibody according to the present invention as a primary antibody and using a labeled antibody labeled with a measurable labeling substance as a secondary antibody is carried out to measure the amount of PSA protein in the separated first complex fraction.
The labeling substance used for labeling the anti-PSA antibody and the method for binding the labeling substance to the antibody, used in the [Method 3'] to [Method 8'], include the same ones as described for the labeling substance used for labeling the anti-PSA antibody and the method of binding the labeling substance to the antibody, in the section (1)-1. In addition, the method for measuring the signal derived from the labeling substance in the complex using the labeled anti-PSA antibody includes the same method as that described in the section (1)-1.
In the [Method 3'] to [Method 8'], the anti-PSA antibody, the anti-free PSA antibody, and the affinity substance with α(2,3) glycan affinity may be immobilized on a solid phase depending on the measurement method.
Examples of the solid phase and the method of immobilizing each antibody to be used on the solid phase, used in the [Method 3'] to [Method 8'], include the same ones as described in the section (1)-1.
In the [Method 3'] to [Method 8'], all of the separation/measurement devices and various reagents that are used

in the art of immunoassays and the like known per se can be used in the method. The types of the affinity substance with α(2,3) glycan affinity and the solid phase used for the measurement are appropriately selected depending on the measurement device to be used and the measurement method to be carried out. Concentrations of anti-PSA antibodies and reagents used for the measurement may be appropriately selected from the concentration ranges commonly used in the related art. The labeling substance used for labeling the anti-PSA antibody is appropriately selected according to the measurement method, the measurement device, and the like for measuring the labeling substance. The labeling substance used for labeling the anti-PSA antibody is appropriately selected according to the measurement method, the measurement device, and the like for measuring the labeling substance. In addition, the measurement conditions and the like (such as reaction temperature, reaction time, pH at the time of reaction, measurement wavelength, and measurement device) in a case of carrying out the measurement may be appropriately selected according to a method known per se.

(2)-2. Method in which value obtained by subtracting amount of free PSA other than α(2,3) free PSA from amount of free PSA in sample is taken as amount of α(2,3) free PSA

In the method of (2)-2, the "amount of free PSA" may be obtained by the method described in the section "(1). Method for measuring amount of free PSA".

In the method of (2)-2, the "amount of free PSA other than α(2,3) free PSA" can be obtained, for example, by the following method.

"A sample is reacted with an affinity substance with α(2,3) glycan affinity to form a complex of α(2,3) free PSA and the affinity substance with α(2,3) glycan affinity. Next, the complex is separated from a solution containing free PSA other than α(2,3) free PSA that did not bind to the affinity substance with α(2,3) glycan affinity. The amount of free PSA other than α(2,3) free PSA is obtained by measuring the amount of free PSA in the separated solution."

That is, α(2,3) free PSA and free PSA other than α(2,3) free PSA can be separated according to the method described in the section (1)-2-1 or (1)-2-2. Then, the amount of free PSA other than the separated α(2,3) free PSA may be measured. In this case, it is not necessary to measure the amount of α(2,3) free PSA.

Specifically, α(2,3) free PSA and free PSA other than α(2,3) free PSA are separated, for example, according to the method of [Method 1] to [Method 8] described in the section (1)-2. Then, the amount of free PSA other than the separated α(2,3) free PSA is measured. The amount of α(2,3) free PSA may be obtained by subtracting the obtained amount of free PSA other than α(2,3) free PSA from the separately measured amount of free PSA in the same sample.

In the method of (2)-2, the method for measuring the amount of free PSA other than α(2,3) free PSA is preferably a method for measuring only the amount of free PSA other than the α(2,3) free PSA separated according to the method of the [Method 3], [Method 4], [Method 6], or [Method 7]. The method for measuring only the amount of free PSA other than the α(2,3) free PSA separated according to the method of [Method 4] is more preferable.

The method of (2)-1 is preferable as the "(2) Method for measuring amount of α(2,3) free PSA". In the method of (2)-1, [Method 3'], [Method 4'], [Method 6'], [Method 7'], [Method 10'], and [Method 11'] are preferable. [Method 4'] and [Method 10'] are more preferable. [Method 4'] is particularly preferable.

In the present invention, in a case of carrying out a measurement method using an affinity substance with α(2,3) glycan affinity, the amount of the affinity substance with α(2,3) glycan affinity used in measuring the amount of α(2,3) free PSA should be sufficient to measure 80% or more of α(2,3) free PSA in the sample.

The amount of such an affinity substance with α(2,3) glycan affinity to be used may be determined taking into account, for example, the binding constant of the affinity substance with α(2,3) glycan affinity for α(2,3) free PSA and the amount of α(2,3) free PSA in a sample.

For example, in a case of using an affinity substance with α(2,3) glycan affinity that has a strong affinity for α(2,3) free PSA and does not re-dissociate in a case where it binds to α(2,3) free PSA, a sufficient amount of the affinity substance with α(2,3) glycan affinity is used such that 80% or more of α(2,3) free PSA in a sample forms a complex with the affinity substance with α(2,3) glycan affinity, at the time of measurement.

For example, in a case of using an affinity substance with α(2,3) glycan affinity that does not have a sufficient affinity for α(2,3) free PSA and re-dissociates even in a case where once it binds to α(2,3) free PSA, it is necessary to use a sufficient amount of the affinity substance with α(2,3) glycan affinity such that 80% or more of α(2,3) free PSA in a sample forms a complex with the affinity substance with α(2,3) glycan affinity, at the time of measurement even after re-dissociation.

The amount of the affinity substance with α(2,3) glycan affinity used to satisfy the conditions as described above is preferably an excess amount (saturated amount) of the affinity substance with α(2,3) glycan affinity with respect to α(2,3) free PSA in a sample.

(3) Method for measuring ratio 1

As a method for obtaining a ratio 1 according to the present invention, for example, there is a "method for obtaining a ratio 1 in which an amount of free PSA and an amount of α(2,3) free PSA in a sample are measured, and the ratio between the obtained amount of free PSA and the obtained amount of α(2,3) free PSA is calculated".
That is, the ratio 1 of the present invention is a "ratio between the amount of free PSA and the amount of α(2,3) free PSA". Hereinafter, it may be simply referred to as "ratio 1".
The ratio 1 may be, for example,

(a) a ratio of amount of α(2,3) free PSA to amount of free PSA (amount of α(2,3) free PSA/amount of free PSA), or
(b) a ratio of amount of free PSA to amount of α(2,3) free PSA (amount of free PSA/amount of α(2,3) free PSA)

It is more preferable to use the ratio of (a), that is, the "ratio of amount of α(2,3) free PSA to amount of free PSA (amount of α(2,3) free PSA/amount of free PSA)" as the ratio 1.
Examples of the method for measuring the amount of free PSA used in the method include the method described in the section "(1) Method for measuring amount of free PSA" in "2. Method for obtaining ratio 1" as described above.
Examples of the method for measuring the amount of α(2,3) free PSA used in the method include the method described in the section "(2) Method for measuring amount of α(2,3) free PSA" in "2. Method for obtaining ratio 1" as described above.
Specific examples of the method for obtaining the ratio 1 include the following method A and method B.
Method A: The amount of α(2,3) free PSA is measured by any of the [Method 1'], [Method 2'], [Method 9], [Method 10], and [Method 11]. Separately, the amount of free PSA is measured by the method described in the section "(1) Method for measuring amount of free PSA". The ratio between the obtained amount of free PSA and the obtained amount of α(2,3) free PSA is obtained to determine the ratio 1.
Examples of the ratio 1 in Method A includes [amount of α(2,3) free PSA/amount of free PSA] and [amount of free PSA/amount of α(2,3) free PSA]. Above all, [amount of α(2,3) free PSA/amount of free PSA] is preferably set to the ratio 1.
In Method A, it is preferred that the amount of free PSA is measured by the method described in the section (1)-1, and the amount of α(2,3) free PSA is measured by [Method 10] or [Method 11]. It is more preferred that the amount of free PSA is measured by the method described in the section (1)-1, and the amount of α(2,3) free PSA is measured by [Method 10],
**[0208]** Method B: The amount of free PSA is measured by any of the [Method 3'] to [Method 8']. The ratio between the obtained amount of α(2,3) free PSA and the [sum of amount of α(2,3) free PSA and amount of free PSA other than α(2,3) free PSA] obtained by any of the [Method 3] to [Method 8] is obtained to determine the ratio 1.
Examples of the ratio 1 in Method B include [amount of α(2,3) free PSA/(amount of α(2,3) free PSA + amount of free PSA other than α(2,3) free PSA)], and [(amount of α(2,3) free PSA + amount of free PSA other than α(2,3) free PSA)/amount of α(2,3) free PSA]. Above all, [amount of α(2,3) free PSA/(amount of α(2,3) free PSA + amount of free PSA other than α(2,3) free PSA)] is preferably set to the ratio 1.
The amount of free PSA and the amount of α(2,3) free PSA to obtain the ratio 1 are preferably measured by any one method of [Method 3] and [Method 3'], [Method 4] and [Method 4'], [Method 5] and [Method 5'], [Method 6] and [Method 6'], [Method 7] and [Method 7'], and [Method 8] and [Method 8'].
Above all, the measurement is more preferably carried out by any one method of [Method 3] and [Method 3'], [Method 4] and [Method 4'], [Method 6] and [Method 6'], and [Method 7] and [Method 7'] and still more preferably [Method 4] and [Method 4'].
Particularly preferred is a method in which both the amount of free PSA and the amount of α(2,3) free PSA are separately measured in one step by the method of the [Method 4] and [Method 4'], and the ratio between the amount of α(2,3) free PSA and the [sum of amount of α(2,3) free PSA and amount of free PSA other than α(2,3) free PSA] is obtained to determine the ratio 1. In the method, particularly preferred is a method in which [amount of α(2,3) free PSA/(amount of α(2,3) free PSA + amount of free PSA other than α(2,3) free PSA)] is set to the ratio 1.
It should be noted that, in a case of obtaining the ratio between the amount of free PSA and the amount of α(2,3) free PSA (ratio 1) in the present invention, it is not necessary to use the amount of free PSA, the amount of α(2,3) free PSA, and the absolute amount (PSA protein amount) of the amount of free PSA other than α(2,3) free PSA. The ratio 1 can also be obtained by using an actual measurement value (for example, a signal value such as intensity of fluorescence or absorbance, a peak area, or a peak height) as the amount of free PSA and the amount of α(2,3) free PSA. The area is preferable as the actual measurement value.
For example, in the method of (1)-2-2 or (2), in a case where α(2,3) free PSA and free PSA other than α(2,3) free PSA are separated (fractionated) by capillary electrophoresis which will be described later, the peak area of each separated fraction may be obtained, and the ratio between [peak area of α(2,3) free PSA fraction] and [peak area of

α(2,3) free PSA fraction + peak area of free PSA fraction other than α(2,3) free PSA] may be set to the ratio 1.

For example,

· [peak area of α(2,3) free PSA fraction/(peak area of α(2,3) free PSA fraction + peak area of free PSA fraction other than α(2,3) free PSA)], or

· [(peak area of α(2,3) free PSA fraction + peak area of free PSA fraction other than α(2,3) free PSA)/peak area of α(2,3) free PSA fraction] may be set to the ratio 1.

The [peak area of α(2,3) free PSA fraction/(peak area of α(2,3) free PSA fraction + peak area of free PSA fraction other than α(2,3) free PSA)] is preferably set to the ratio 1.

In addition, for example, in a case where α(2,3) free PSA and free PSA other than α(2,3) free PSA are separated (fractionated), and each thereof is detected using a fluorescently labeled anti-PSA antibody, for example, the ratio obtained by the following method may be set to the ratio 1.

· [amount of fluorescence of α(2,3) free PSA fraction/(amount of fluorescence of α(2,3) free PSA fraction) + amount of fluorescence of free PSA fraction other than α(2,3) free PSA)], or

· [(amount of fluorescence of α(2,3) free PSA fraction + amount of fluorescence of free PSA fraction other than α(2,3) free PSA)/amount of fluorescence of α(2,3) free PSA fraction]

It is preferable to use the [amount of fluorescence of α(2,3) free PSA fraction/(amount of fluorescence of α(2,3) free PSA fraction) + amount of fluorescence of free PSA fraction other than α(2,3) free PSA)] as the ratio 1.

As one embodiment of the method for obtaining the ratio 1, a method in which a lectin is used as the affinity substance with α(2,3) glycan affinity, and the amount of free PSA and the amount of α(2,3) free PSA are measured by the method of [Method 4] and [Method 4'] to obtain the ratio 1 will be described below as an example.

1) A sample, a labeled first antibody in which an anti-PSA antibody is labeled with a labeling substance, a second antibody that is an anti-PSA antibody, and a lectin having an affinity for a glycan in which the terminal sialic acid residue of the glycan is α(2,3)-linked to the second galactose residue from the terminal of the glycan (affinity substance with α(2,3) glycan affinity) are brought into contact with one another to form a complex of the labeled first antibody, α(2,3) free PSA, the second antibody, and the lectin (first complex), and a complex of the labeled first antibody, free PSA other than α(2,3) free PSA, and the second antibody (second complex).

2) The first complex and the second complex obtained in the step of 1) are separated (fractionated).

3) The signal derived from the labeling substance of the labeled first antibody constituting the first complex and the second complex is measured, whereby the amount of the first complex and the amount of the second complex separated in the step of 2) are measured.

4) A sum (amount of free PSA) of the amount of the first complex and the amount of the second complex obtained in the step of 3) is obtained, and the ratio between the sum and the amount of the first complex obtained in the step of 3) is obtained, whereby the ratio 1 between the amount of free PSA and the amount of α(2,3) free PSA is obtained.

At least one of the first antibody or the second antibody is an anti-free PSA antibody. The first antibody is preferably an anti-free PSA antibody.

The first complex and the second complex may be separated on the basis of the affinity of the lectin for an α(2,3) glycan or may be separated on the basis of the difference in mass, charge, size, or the like between the first complex and the second complex (for example, electrophoresis).

In addition, the ratio 1 between the amount of the free PSA and the amount of the α(2,3) free PSA obtained in the step of 4) is preferably the [amount of first complex/(amount of first complex + amount of second complex)] = [amount of α(2,3) free PSA/amount of free PSA].

In the measurement using the affinity substance with α(2,3) glycan affinity, the step of separating (fractionating) α(2,3) free PSA bound to the affinity substance with α(2,3) glycan affinity, and free PSA other than α(2,3) free PSA not bound to the affinity substance with α(2,3) glycan affinity can be carried out, for example, by capillary electrophoresis, surface plasmon resonance method, lectin microarray, or immunoassay. Among them, capillary electrophoresis, surface plasmon resonance method, or immunoassay is preferable, and capillary electrophoresis is more preferable.

Hereinafter, a specific example of the method in which the amount of α(2,3) free PSA and the amount of free PSA are measured by each of the methods using the affinity substance with α(2,3) glycan affinity, and the ratio 1 is obtained will be described.

· Capillary electrophoresis

As the method of obtaining the amount of α(2,3) free PSA by capillary electrophoresis and obtaining the ratio between the amount of free PSA and the amount of α(2,3) free PSA (ratio 1), for example, first, a sample containing PSA is brought into contact with and reacted with a labeled anti-free PSA antibody in which an anti-free PSA antibody according to the present invention is labeled with a labeling substance, a [labeled anti-free PSA antibody-α(2,3) free PSA] complex and a [labeled anti-free PSA antibody-free PSA other than α(2,3) free PSA] complex in the obtained reaction solution are separated by carrying out capillary electrophoresis in the presence of an affinity substance with α(2,3) glycan affinity, and then the amount of the labeling substance derived from the [labeled anti-free PSA antibody-α(2,3) free PSA] (first complex) and the amount of the labeling substance derived from the [labeled anti-free PSA antibody-free PSA other than α(2,3) free PSA] (second complex) are measured. The amount of free PSA in the sample is a sum of the amounts of labeling substances of the first complex and the second complex. Then, the ratio between the obtained amount of free PSA and the obtained amount of α(2,3) free PSA is obtained.

Since the amount of the labeling substance derived from the first complex and the amount of the labeling substance derived from the second complex correspond to the respective peak area values obtained by capillary electrophoresis, the ratio may be obtained using the respective peak area values.

Specific examples of the anti-free PSA antibody and the labeling substance used in the method are as described above.

The solvent for reacting the sample with the labeled anti-free PSA antibody may be, for example, a buffer solution. The buffer solution to be used for this purpose is not particularly limited as long as it is commonly used in the art, but those having a buffering action in the vicinity of neutral pH, usually pH 5.0 to 10.0, preferably pH 6.5 to 8.5, can be mentioned. Specific examples of the buffer solution include a Tris-HCl buffer solution, a MES buffer solution, a HEPES buffer solution, a borate buffer solution, a phosphate buffer solution, a veronal buffer solution, and a Good's buffer solution. In addition, the concentration of a buffering agent in these buffer solutions is appropriately selected from the range of usually 5 to 1,000 mM and preferably 5 to 300 mM.

The buffer solution may further contain a sensitizer, a surfactant, a preservative (for example, sodium azide, salicylic acid, or benzoic acid), a stabilizer (for example, albumin, globulin, water-soluble gelatin, surfactant, or saccharides), an activator agent, and others which are used in the art and which do not inhibit the stability with coexisting reagents or do not inhibit an antigen-antibody reaction. In addition, concentration ranges and the like of these reagents and the like may be appropriately selected and used in a concentration range commonly used in the measurement method known per se.

In a case where a commercially available kit dedicated to a capillary electrophoresis apparatus is used, a buffer solution included in the kit may be used.

The concentration of the antibody in the solution containing the labeled anti-free PSA antibody may be any concentration as long as it is within the target concentration range in a case of mixing the sample and the solution containing the labeled anti-free PSA antibody. For example, the concentration of the antibody in the solution containing the labeled anti-PSA antibody may be 0.1 to 200 μM, preferably 0.5 to 50 μM, more preferably 0.5 to 20 μM, and still more preferably 0.5 to 10 μM. That is, the lower limit value thereof is 0.1 μM and preferably 0.5 μM. In addition, the upper limit value thereof is 200 μM, preferably 50 μM, more preferably 20 μM, and still more preferably 10 μM.

The concentration of the labeled anti-free PSA antibody according to the present invention in the reaction solution in a case of contacting/reacting the sample with the labeled anti-free PSA antibody according to the present invention varies depending on the concentration of PSA in the sample and is not particularly limited, but it is usually 0.1 to 1,000 nM, preferably 0.1 to 500 nM, and more preferably 0.5 to 200 nM. That is, the lower limit value thereof is 0.1 nM and preferably 0.5 nM. In addition, the upper limit value thereof is 1,000 nM, preferably 500 nM, and more preferably 200 nM.

In addition, the pH during the reaction between the sample and the labeled anti-free PSA antibody according to the present invention is not particularly limited as long as it does not inhibit an antigen-antibody reaction, and it is in the range of usually 6.0 to 10.0 and preferably 6.0 to 8.0. The temperature during the reaction is not particularly limited as long as it does not inhibit an antigen-antibody reaction, and it is in the range of usually 10°C to 50°C and preferably 20°C to 40°C. In addition, the reaction time varies depending on the antibody according to the present invention to be used and reaction conditions such as pH and temperature, so that the reaction may be carried out for about 1 to 60 minutes and preferably 1 to 15 minutes depending on each.

After the reaction is completed, the first complex and the second complex in the obtained reaction solution are separated by carrying out capillary electrophoresis in the presence of an affinity substance with α(2,3) glycan affinity, and the amounts of the first complex and the second complex are measured.

In the present invention, among the capillary electrophoresis, it is preferable to carry out the electrophoresis which is carried out by a capillary chip or a microcapillary chip. The microchip capillary electrophoresis is a technique of forming a capillary having a cross section with a diameter of 100 μm or less on a chip substrate and carrying out

electrophoresis in this capillary, and is a method of separating a substance utilizing the difference in charge of the substance present in a sample as a difference in its mobility by applying a voltage to the capillary.

Capillary electrophoresis is classified into capillary zone electrophoresis and capillary gel electrophoresis depending on the electrophoretic solution to be used, but the method of the present invention can be applied to any of them. In view of the accuracy of separation, capillary gel electrophoresis is preferable among the capillary electrophoresis.

The electrophoretic solution used in the capillary electrophoresis is not particularly limited as long as it is commonly used in the related art.

The affinity substance with α(2,3) glycan affinity may be contained in the electrophoretic solution. However, during the separation by capillary electrophoresis, it is preferred that the affinity substance with α(2,3) glycan affinity is at a higher concentration than the amount at which the α(2,3) free PSA and the affinity substance with α(2,3) glycan affinity can be completely bound.

For example, in a case where the affinity substance with α(2,3) glycan affinity is a lectin, the concentration thereof is 0.1 mg/mL to 20 mg/mL, preferably 1.0 mg/mL to 10 mg/mL, and more preferably 2.0 mg/mL to 5.0 mg/mL. In a case where the affinity substance with α(2,3) glycan affinity is an antibody, the concentration thereof is 0.01 mg/mL to 20 mg/mL, preferably 0.05 mg/mL to 10 mg/mL, and more preferably 0.1 mg/mL to 5.0 mg/mL.

The concentration of the affinity substance with α(2,3) glycan affinity in the microchannel may be 0.1 mg/mL to 20 mg/mL.

In a case of separating a complex by capillary electrophoresis, it is preferred that any antibody used in the measurement system, such as an anti-PSA antibody or an antibody as an affinity substance with α(2,3) glycan affinity, is labeled with a charged carrier molecule such as an anionic substance, even in a case where a lectin is used as the affinity substance with α(2,3) glycan affinity or an antibody is used as the affinity substance with α(2,3) glycan affinity. Specifically, the antibody is preferably labeled with, for example, a nucleic acid chain. In this case, the antibody may be labeled with both a charged carrier molecule and the labeling substance for detection. In addition, for example, an anti-free PSA antibody labeled with a labeling substance for detection and an anti-PSA antibody labeled with a charged carrier molecule may be used.

Examples of the type and length of the nucleic acid chain used for such purpose and the method of binding the nucleic acid chain to the antibody include known nucleic acid chain types and lengths and methods disclosed in, for example, JP4214779B and JP4862093B.

In addition, after a reactive functional group is introduced in advance into the nucleic acid chain, the reactive functional group-introduced nucleic acid chain may be bound to the antibody according to the present invention by the method described in JP4214779B. As a method of introducing the reactive functional group into the nucleic acid chain, a method known per se can be mentioned.

In addition, a reactive functional group can be introduced into the nucleic acid terminus, for example, by a method in which PCR is carried out using a PCR primer in which a reactive functional group is introduced at the 5' terminal, thereby obtaining a nucleic acid chain having a reactive functional group introduced at the 5' terminal as a PCR product (Molecular Cloning: A Laboratory Manual Second Edition, J. Sambrook, E. F. Fritsch, T. Maniatis, Cold Spring Harbor Laboratory Press, or the like) as a method of binding the nucleic acid chain and the antibody.

A solution for dissolving a sample, an antibody or the like to be subjected to capillary electrophoresis, the type of electrophoretic solution, additives, and specific conditions for capillary electrophoresis may be according to a method known per se. For example, in a case where capillary electrophoresis is used, it may be carried out according to the method described in J. Chromatogr. 593, 253-258 (1992), Anal. Chem. 64, 1926-1932 (1992), WO2007/027495A, JP4214779B, and the like.

In a case where capillary electrophoresis is carried out by an automatic analyzer, capillary electrophoresis may be carried out according to the method described in the instruction manual attached to the apparatus under the conditions described in the instruction manual.

As a specific example of the method for measuring α(2,3) free PSA by capillary electrophoresis, given below is a method in which, using MAA as an affinity substance with α(2,3) glycan affinity and using a labeled first antibody in which an anti-free PSA antibody is labeled with a fluorescent substance, and using a second antibody in which an anti-PSA antibody is labeled with DNA, microchip capillary electrophoresis utilizing lectin affinity is carried out to separate PSA on the basis of the degree of affinity of lectin for the glycan of PSA, and the separated PSA is measured with a fluorescence detector.

That is, 1 to 50 μL of a sample containing PSA is reacted with a test solution containing a fluorescently labeled anti-free PSA antibody in amount of usually 0.001 to 10 μM and preferably 0.01 to 1 μM.

The sample used for capillary electrophoresis may be a sample collected from a living body or a sample prepared by subjecting a sample collected from a living body to desalting and various purification steps.

The obtained reaction solution, 2 to 50 μL of a test solution containing a DNA-labeled anti-PSA antibody in the amount of usually 0.001 to 10 μM, preferably 0.01 to 1 μM, a electrophoresis buffer solution, and an internal standard substance (for example, fluorescent substance: HiLyte 647 (manufactured by AnaSpec, Inc.) or the like) are introduced

into a capillary of, for example, an internal diameter of 5 to 500 μm, preferably 50 to 200 μm, and more preferably 50 to 100 μm, and a length of 1 to 10 cm by a pressure method of 1 to 10 psi for 30 to 60 seconds. The reaction is carried out for 5 seconds to 30 minutes and preferably 10 seconds to 15 minutes while keeping the temperature at 20°C to 40°C. The [fluorescently labeled anti-free PSA antibody-α(2,3) free PSA-DNA-labeled anti-PSA antibody] complex and the [fluorescently labeled anti-free PSA antibody-free PSA other than α(2,3) free PSA-DNA-labeled anti-PSA antibody] complex obtained are separated by carrying out electrophoresis with application of a voltage of 1,000 to 5,000 V for 10 seconds to 60 minutes in the presence of MAA (0.1 mg/mL to 20 mg/mL). Then, the electrophoretic state of the complex is measured with a detector such as a fluorescence detector or a UV detector to obtain an electropherogram.

The peak of α(2,3) free PSA (containing a [fluorescently labeled anti-free PSA antibody]-[α(2,3) free PSA]-[DNA-labeled anti-PSA antibody] complex), and the peak of other free PSA peak (containing a [fluorescently labeled anti-free PSA antibody]-[free PSA other than α(2,3) free PSA]-[DNA-labeled anti-PSA antibody] complex) can be distinguished from their electrophoretic positions. Therefore, the amount of α(2,3) free PSA in the sample is taken as the peak area of the peak. In addition, the sum of the peak area of α(2,3) free PSA and the peak area of free PSA other than α(2,3) free PSA obtained is taken as the amount of free PSA.

As described above, in a case where capillary electrophoresis is used, the amount of free PSA and the amount of α(2,3) free PSA can be measured at the same time in the same sample.

The ratio 1 can be obtained by obtaining the ratio of the peak areas obtained, that is

· [peak area of α(2,3) free PSA fraction/(peak area of α(2,3) free PSA fraction + peak area of free PSA fraction other than α(2,3) free PSA)], or

· [(peak area of α(2,3) free PSA fraction + peak area of free PSA fraction other than α(2,3) free PSA)/peak area of α(2,3) free PSA fraction] may be set to the ratio 1.

The [peak area of α(2,3) free PSA fraction/(peak area of α(2,3) free PSA fraction + peak area of free PSA fraction other than α(2,3) free PSA)] is more preferably used as the ratio 1.

The capillary electrophoresis may be carried out using a commercially available fully automated measurement device. For example, μTAS Wako i30 (manufactured by FUJIFILM Wako Pure Chemical Corporation) can be mentioned.

· Surface plasmon resonance method

The surface plasmon resonance method is an intermolecular interaction analysis system that analyzes the interactions between biomolecules using the optical phenomenon of so-called surface plasmon resonance (SPR) which occurs in a case where surface plasmons are excited at the metal/liquid interface. The surface plasmon resonance method uses a surface plasmon resonance spectrometer to detect trace amounts of mass change occurring on the surface of the sensor chip due to binding and dissociation between two molecules as SPR signals. Since interactions between biomolecules are monitored in real time, kinetics information indicating that bonding/dissociation between biomolecules is fast or slow is obtained.

As a representative analysis system of the surface plasmon resonance method, there is a Biacore™ method. The Biacore method is commonly simply referred to as Biacore. In addition, in a case of "Biacore", it sometimes refers to a Biacore device used for Biacore's analysis system.

The measurement by a surface plasmon resonance method may be carried out under optimum conditions for the measurement according to the attached instruction manual.

Examples of the method for obtaining the ratio 1 by surface plasmon resonance method include the following methods.

That is, the anti-free PSA antibody is immobilized on the surface of the sensor chip. In a case where detection light is projected from the backside of the sensor chip such that the light is totally reflected at the boundary surface between the gold thin film of the sensor chip and the glass, a portion having a reduced reflection intensity appears in part of the reflected light. The angle at which the dark portion of this light appears depends on the refractive index of the solvent on the surface of the sensor chip. Next, a sample is flowed from the flow channel of the surface plasmon resonance spectrometer to the surface of the sensor chip. In a case where the anti-free PSA antibody immobilized on the gold thin film surface binds to the PSA molecule contained in the sample flowing through the flow channel, the mass of the molecule immobilized on the gold thin film surface increases, and therefore the refractive index of the solvent changes. At this time, the position of the dark portion of the reflected light shifts according to the change of the mass. Conversely, in a case where molecules are dissociated, the position of the dark portion returns from the shifted position. The shift angle represents the mass change on the surface of the sensor chip. In the surface plasmon resonance method, the intermolecular bonding/dissociation is monitored from this angle change. On the basis of the obtained results, first, the amount of free PSA in a sample can be measured.

Next, the solution containing an affinity substance with α(2,3) glycan affinity is flowed from the flow channel of

the surface plasmon resonance spectrometer to the surface of the sensor chip. Similarly, in a case where the PSA of [anti-free PSA antibody-free PSA] which is a complex formed on the surface of the gold thin film binds to the affinity substance with α(2,3) glycan affinity flowing through the flow channel, the refractive index of the solvent changes in the same manner, so that the intermolecular bonding/dissociation is monitored in the same manner. On the basis of the obtained results, it is possible to measure the amount of α(2,3) free PSA in the sample.

The ratio between the obtained amount of free PSA and the obtained amount of α(2,3) free PSA (ratio 1) in the sample is obtained.

It is more preferable to use the [amount of α(2,3) free PSA/amount of free PSA] as the ratio 1.

Another method for obtaining the ratio 1 by surface plasmon resonance method may be, for example, the following method.

That is, using one or more reference liquids whose ratio between the amount of α(2,3) free PSA and the amount of free PSA is known, prepared in advance using an α(2,3) free PSA standard, the measurement by the surface plasmon resonance method is carried out to obtain each sensorgram. Next, the measurement is similarly carried out using a test sample to obtain a sensorgram. The ratio between the amount of free PSA and the amount of α(2,3) free PSA (ratio 1) in the test sample is obtained by comparing the sensorgram obtained using the reference liquid with the sensorgram obtained using the test sample.

It is preferred that the [amount of α(2,3) free PSA/amount of free PSA] is set to the ratio 1.

As the reference liquid used in the method, one having a numerical value at which the ratio of the amount of α(2,3) free PSA to the amount of free PSA will serve as an index in the subsequent determination of prostate carcinoma is used. For example, a reference liquid having the ratio of 25%, 45%, or 50% may be used.

As a method for preparing the reference liquid, for example, the following methods can be mentioned.

That is, the α(2,3) free PSA standard and the α(2,6) free PSA standard are dissolved in appropriate buffer solutions, respectively, and adjusted so that the protein amounts of the respective solutions are the same. Then, a solution of α(2,3) free PSA standard is diluted with a solution of α(2,6) free PSA standard and adjusted to the target ratio, whereby a reference liquid can be obtained.

Details of the method for constructing the α(2,3) free PSA standard and the α(2,6) free PSA standard are as described in the section (1)-2-1.

· Lectin microarray method

A lectin microarray method developed by Glycomedicine Technology Research Center, National Institute of Advanced Industrial Science and Technology, or the like can also be used in the present invention.

The lectin microarray is an array in which several tens of lectins having different specificities for glycans are arranged and immobilized in a spot shape on a slide glass. In addition, the evanescent field refers to a state where weak light exudes from the substrate (slide glass) interface. After allowing the fluorescently labeled glycoprotein to interact with the lectin microarray, excitation light is irradiated to the slide glass to generate an evanescent field. Since the excitation light reaches about 100 nm to 200 nm from the interface and the glycan bound to the lectin also exists between 100 nm and 200 nm from the interface, only the glycan bound to the lectin microarray can emit light. With this technique, the fluorescence of the lectin microarray can be detected without carrying out the washing operation. After allowing the glycoprotein without fluorescent labeling to interact with the lectin microarray, the antibody against the core protein of the glycoprotein may be reacted with the fluorescently labeled fluorescent antibody and then the fluorescence may be detected by the same method as described above.

The measurement with a lectin microarray may be carried out according to the protocol described in, for example, MICROARRAY METHODS AND PROTOCOLS (CRC Press), edited by Robert S. Matson, "Chapter 9: Lectin Microarrays", Masao Yamada, p. 141, 2009.

For example, the following methods can be mentioned as the method of obtaining the amount of α(2,3) free PSA by the lectin microarray method and obtaining the ratio between the amount of free PSA and the amount of α(2,3) free PSA (ratio 1),.

A microarray in which a lectin which is the affinity substance with α(2,3) glycan affinity according to the present invention and a plurality of lectins having an affinity for a glycan of PSA other than an α(2,3) glycan are immobilized is constructed. The desired microarray may be prepared in accordance with the method described in, for example, Kuno A. et al., Nat Methods. 2005 Nov, vol. 2, No. 11, pp. 851 to 856. Alternatively, a commercially available microarray (LecChip™, manufactured by GlycoTechnica Ltd.) or the like may be used.

A sample pretreated as necessary and a fluorescently labeled anti-free PSA antibody are added dropwise into the microarray and allowed to react to form a [fluorescently labeled anti-free PSA antibody-α(2,3) free PSA-lectin] complex on the microarray. Next, the fluorescence derived from the fluorescently labeled anti-free PSA antibody is measured using an evanescent wave excitation fluorescence scanner. By carrying out the measurement in the same manner using an α(2,3) free PSA standard of known concentration and conversion into quantitative values on the basis of the obtained

measurement value, the amount of α(2,3) free PSA in the sample is obtained.

The ratio between the separately obtained amount of free PSA and the amount of α(2,3) free PSA measured by the microarray method (ratio 1) is obtained.

It is preferred that the [amount of α(2,3) free PSA/amount of free PSA] is set to the ratio 1.

The method of measuring the amount of free PSA is as described in the section "(1) Method of measuring amount of free PSA".

· Immunoassay

The following [Method a] and [Method b] can be mentioned as the method of obtaining the amount of α(2,3) free PSA by common immunoassay and obtaining the ratio between the amount of free PSA and the amount of α(2,3) free PSA (ratio 1).

[Method a]

An affinity substance with α(2,3) glycan affinity is immobilized on a solid phase. A sample is brought into contact with and reacted with the solid phase. After washing the solid phase, a labeled anti-free PSA antibody in which an anti-free PSA antibody is labeled with a detectable labeling substance is brought into contact with and reacted with the solid phase to produce a complex of the affinity substance with α(2,3) glycan affinity, α(2,3) free PSA, and the labeled anti-free PSA antibody on the solid phase. The unreacted labeled anti-free PSA antibody is removed by washing or the like, and then the amount of the labeling substance is measured by a measurement method corresponding to the labeling substance of the labeled anti-free PSA antibody. On the basis of the obtained measurement results, the amount of α(2,3) free PSA is obtained by conversion into quantitative values by a conventional method using the results obtained by carrying out the measurement using an α(2,3) free PSA standard of known concentration in advance. The amount of free PSA in the same sample is obtained by the method for separately measuring the amount of free PSA in a sample as described above. The ratio between the obtained amount of free PSA and the obtained amount of α(2,3) free PSA (ratio 1) is obtained.

It is preferred that [amount of α(2,3) free PSA/amount of free PSA] is set to the ratio 1.

[Method b]

An anti-free PSA antibody is immobilized on a solid phase. A sample is brought into contact with and reacted with the solid phase. After washing the solid phase, a labeled affinity substance with α(2,3) glycan affinity in which an affinity substance with α(2,3) glycan affinity is labeled with a detectable labeling substance is brought into contact with and reacted with the solid phase to produce a complex of the anti-free PSA antibody, α(2,3) free PSA, and the labeled affinity substance with α(2,3) glycan affinity on the solid phase. The unreacted labeled affinity substance with α(2,3) glycan affinity is removed by washing or the like, and then the labeling substance is measured by a method corresponding to the labeling substance of the labeled affinity substance with α(2,3) glycan affinity. On the basis of the obtained measurement results, the amount of α(2,3) free PSA is obtained by conversion into quantitative values by a conventional method using the results obtained by carrying out the measurement using an α(2,3) free PSA standard of known concentration in advance. The amount of free PSA in the same sample is obtained by the method for separately measuring the amount of free PSA in a sample as described above. The ratio between the obtained amount of free PSA and the obtained amount of α(2,3) free PSA (ratio 1) is obtained.

It is preferred that [amount of α(2,3) free PSA/amount of free PSA] is set to the ratio 1.

In the immunoassay, examples of the insoluble carrier used as the solid phase, the free PSA antibody, and the method of immobilizing the antibody on the insoluble carrier include the same as those described in the section "(1)-1. Method for directly measuring amount of free PSA in sample". In addition, the types and concentrations of use of various reagents used in the measurement, the measurement conditions for carrying out the measurement (such as a reaction temperature, a reaction time, a pH at the time of reaction, a measurement wavelength, and a measurement device), and the like may be set in accordance with a measurement operation such as immunoassay known per se.

In the method, the labeling substance, the solid phase, the anti-free PSA antibody, the method for measuring the labeling substance, the measurement conditions, and other details are the same as described above.

· Mass spectrometry

As another method for obtaining the ratio 1 according to the present invention, there is a method using a method for measuring an amount of α(2,3) free PSA without using an affinity substance with α(2,3) glycan affinity. For example, mass spectrometry using a multistage tandem mass spectroscope or a high performance liquid chromatograph mass

spectrometer can be mentioned.

An example of a method for obtaining the ratio 1 by mass spectrometry is shown below.

First, using a solid phase on which an anti-free PSA antibody is immobilized, free PSA in a sample is isolated by a conventional method. Then, the glycan structure of the isolated free PSA is analyzed using, for example, a high performance liquid chromatograph mass spectrometer. By this method, the amount of α(2,3) free PSA and the amount of free PSA other than α(2,3) free PSA in the sample can be measured without using the affinity substance with α(2,3) glycan affinity. Based on the obtained measurement values, a ratio between the amount of α(2,3) free PSA and the [amount of α(2,3) free PSA and amount of free PSA other than α(2,3) free PSA] (ratio 1) is obtained. It is preferred that the [amount of α(2,3) free PSA/(amount of α(2,3) free PSA + amount of free PSA other than α(2,3) free PSA)] is set to the ratio 1.

<3. Method for measuring volume of prostate >

The prostate volume (PV) can be measured by a non-invasive method such as common ultrasonic examination or magnetic resonance imaging (MRI) examination. It is preferable to carry out by ultrasonic examination.

The method of ultrasonic examination is not limited in any way. Usually, the ultrasonic examination may be carried out by either applying an apparatus that emits ultrasonic waves through the lower abdomen of a subject or inserting the apparatus through the anus of the subject.

Examples of the apparatus that emits ultrasonic waves include, but are not limited to, ultrasound diagnostic apparatuses such as general-purpose ultrasound diagnostic imaging apparatuses that are commonly used in clinical settings.

The prostate volume may be measured by any method of measuring a volume of the prostate, including conventional methods well known to those of skill in the art (such as the TRUS test using the expression H × W × L × 0.52). For example, the prostate is visualized by ultrasonic examination to measure the size (volume) of the prostate. Usually, the volume of the prostate is automatically calculated by the diagnostic software attached to the ultrasound diagnostic imaging apparatus.

<4. Method for obtaining ratio 2>

The ratio 2 according to the present invention is a ratio between the ratio 1 obtained by the method described in the section "2. Method for obtaining ratio 1" and the volume of the prostate of the same subject as the one from which the sample used to obtain the ratio 1 was taken.

That is, the ratio 2 includes the following four cases.

(i) [amount of α(2,3) free PSA/amount of free PSA]/[volume of prostate],
(ii) [amount of free PSA/amount of α(2,3) free PSA]/[volume of prostate],
(iii) [volume of prostate]/[amount of α(2,3) free PSA/amount of free PSA], and
(iv) [volume of prostate]/[amount of free PSA/amount of α(2,3) free PSA].

Among the above, the ratio 2 obtained by (i) is particularly preferable.

An example of the method for obtaining the ratio 2 in a case where the ratio 1 and the ratio 2 are as follows (in a case of (i) above) will be described.

Ratio 1 = [amount of α(2,3) free PSA/amount of free PSA]
Ratio 2 = [ratio 1/volume of prostate]

In a case where the ratio 1 is 40% and the volume of the prostate is 50 $cm^3$, the ratio 2 is as follows.

$$\text{Ratio 2} = [\text{amount of } \alpha(2,3) \text{ free PSA/amount of free PSA}]/[\text{volume of prostate}]$$
$$= 0.80$$

<5. Method for determining prostate carcinoma>

The method for determining prostate carcinoma according to the present invention is "a method including obtaining a ratio 1 between an amount of a free PSA and an amount of an α(2,3) free PSA in a sample derived from a subject; obtaining a ratio 2 between the ratio 1 and a volume of the prostate of the subject; and determining prostate carcinoma based on the obtained ratio 2".

That is, the ratio 2 is obtained by the method described in the section "4. Method for obtaining ratio 2". Based on the result, data (information such as ratio 2 value, comparison between ratio 2 value and cutoff value, and degree of increase of ratio 2) is obtained for determining prostate carcinoma using the ratio 2 as an index.

The determination (diagnosis/test) of prostate carcinoma is carried out using the obtained data.

An example of the determination method in a case of using (i) [amount of $\alpha$(2,3) free PSA/amount of free PSA]/[volume of prostate] as the ratio 2 will be described below.

In a case where the ratio 2 obtained using a sample derived from a subject is equal to or higher than a predetermined cutoff value (reference value), it is possible to determine that the subject who provided the sample has prostate carcinoma (positive for prostate carcinoma), or is highly likely to have the same. In a case where the ratio 2 is less than the cutoff value, it is possible to determine that the subject does not have prostate carcinoma (negative for prostate carcinoma) or is unlikely to have the same.

In addition, there is a method of setting a plurality of determination categories corresponding to the cutoff value of the ratio 2 or a range of that cutoff value and making a determination. For example, the determination categories are set to [(1) There is no probability of prostate carcinoma, (2) There is a low probability of prostate carcinoma, (3) There is a sign of prostate carcinoma, (4) There is a high probability of prostate carcinoma, and the like]. Then, it is possible to carry out the determination of prostate carcinoma by determining which determination category the value of the ratio 2 of the subject-derived sample falls into.

In addition, it is possible to diagnose the degree of progression or malignancy of prostate carcinoma or it is possible to make a post-operative prognosis in such a manner that, in the same subject, the value of the ratio 2 of the subject-derived sample obtained at a certain time point is compared with the value of the ratio 2 obtained at a different time point, and whether the value of the ratio 2 has increased or decreased is evaluated and/or the degree of increase or decrease of the value of the ratio 2 is evaluated.

That is, in a case where a test result is obtained that an increase in the value of the ratio 2 is recognized, it can be determined that the pathological condition has progressed to prostate carcinoma (or the malignancy of prostate carcinoma has increased), or that there is a sign of progression of the pathological condition to prostate carcinoma (or there is a sign that the malignancy of prostate carcinoma is increased).

In addition, in a case where a test result is obtained that no change in the ratio 2 is recognized, it can be determined that there is no change in the pathological condition of prostate carcinoma.

Furthermore, in a case where a test result is obtained that the decrease of the ratio 2 is recognized, it can be determined that the pathological condition of prostate carcinoma is improved.

For the cutoff value (reference value), a ratio 2 is obtained by the measurement method using a prostate carcinoma patient-derived sample and a non-cancerous subject-derived sample. Then, the cutoff value may be set based on a boundary value between the values of the ratio 2 for a prostate carcinoma patient and the ratio 2 for a non-cancerous subject. The average value of the ratio 2 of a non-cancerous subject may be set as the cutoff value.

The "non-cancerous subject" in the present invention means the "non-prostate carcinoma subject" who is confirmed not to have prostate carcinoma. The "non-cancerous subject" may be a healthy subject or a patient with prostatic hypertrophy or the like.

In addition, the cutoff value may be obtained by an analysis using a Relative Operating Characteristic curve (ROC curve), for example, by a conventional method.

As an example of the method for obtaining the cutoff value by the analysis using the ROC curve, the following method can be mentioned.

First, based on the obtained ratio 2, an ROC curve is created by a conventional method.

Next, as usual, a straight line with an angle of 45 degrees in contact with the ROC curve is drawn, and an intersection point with that straight line, that is, a value at which maximizes the "sensitivity % - (100 - specificity %)" is obtained. That value may be used as the cutoff value.

Specific examples of the case of determining prostate carcinoma by the method of the present invention using the (i) [amount of $\alpha$(2,3) free PSA/amount of free PSA]/[volume of prostate] as the ratio 2 are shown below.

[Example 1]

(1) A ratio 2 is obtained using a sample derived from a non-cancerous subject.
(2) A ratio 2' is obtained using a sample derived from a subject.
(3) The ratio 2' is compared with the ratio 2, and in a case where the ratio 2' is equal to or higher than the ratio 2, it is determined that the subject who provided the sample has prostate carcinoma (positive for prostate carcinoma), or is highly likely to have the same.

[Example 2]

(1) A ratio 2 is obtained using a sample derived from a non-cancerous subject, and a cutoff value of the ratio 2 is set for determining prostate carcinoma based on the ratio 2.
(2) The ratio 2 is obtained using a sample derived from a subject.
(3) The ratio 2 is compared with the cutoff value set in (1) above, and in a case where the ratio 2 is equal to or higher than the cutoff value, it is determined that the subject who provided the sample has prostate carcinoma (positive for prostate carcinoma), or is highly likely to have the same.

It should be noted that in a case where an appropriate cutoff value is set, it is not necessary to set the cutoff value again each time the subject is subjected to prostate carcinoma determination.
In a case where it is determined that the patient who is a subject has prostate carcinoma by the method for determining prostate carcinoma according to the present invention, an appropriate treatment of prostate carcinoma can be given to the subject.
In addition, in a case where it is determined that the patient who is a subject may have or is highly likely to have prostate carcinoma by the method for determining prostate carcinoma according to the present invention, it is possible to choose to carry out a tissue examination (biopsy). In addition, in a case where it is determined that the patient does not have or is unlikely to have prostate carcinoma, it is possible to select a therapeutic strategy in which biopsy is not carried out and follow-up is carried out as necessary.

<6. Biopsy avoidance rate>

The probability of avoiding unnecessary biopsies is indicated by a "biopsy avoidance rate". The "biopsy avoidance rate" refers to "specificity while maintaining a diagnostic sensitivity of 90%".
For example, a case where the cutoff value is 0.8 and the biopsy avoidance rate is 55% suggests that, in cases where the cutoff value is 0.8 or less, the percentage that can be diagnosed (confirmed) as negative without a biopsy is 55%. In other words, the method for determining prostate carcinoma with a cutoff value of 0.8 means that, assuming that the diagnostic sensitivity for prostate carcinoma is 90%, an unnecessary biopsy can be avoided by 55% for subjects who do not have prostate carcinoma.
Conventional methods for determining prostate carcinoma using the total PSA value as an index are remarkably lacking in specificity. Therefore, even patients with negative biopsy findings needed repeated biopsies to rule out the possibility of prostate carcinoma. As a result, there is a problem that many unnecessary biopsies are carried out even though the biopsy has a risk of infection.
On the other hand, the method for determining prostate carcinoma according to the present invention can determine prostate carcinoma with high specificity and high sensitivity and by non-invasive means.
In addition, since prostate carcinoma can be determined with a high biopsy avoidance rate, the number of subjects requiring biopsy can be reduced. In other words, the determination according to the method for determining prostate carcinoma according to the present invention can avoid an unnecessary biopsy for a subject who does not have prostate carcinoma.
In addition, prostate carcinoma includes benign prostate carcinoma and malignant prostate carcinoma, and in a case of benign prostate carcinoma, the progression thereof is slow, so there is also an option to follow up without an invasive treatment such as surgery. On the other hand, since malignant prostate carcinoma progresses rapidly, it is necessary to find prostate carcinoma at an early stage and determine the malignancy thereof. In addition, in a case where the malignancy of prostate carcinoma can be determined, it will be an important guideline in setting a therapeutic strategy for prostate carcinoma.
The D'Amico classification is used as a method for estimating and evaluating the probability of recurrence of prostate carcinoma which is related to the malignancy of prostate carcinoma, and the prognosis of life. As a result of comparing the prostate carcinoma patients after being subjected to the method for determining prostate carcinoma according to the present invention and classification by the D'Amico classification, patients with a low value of ratio 2 according to the present invention tended to be classified into a low risk group by the D'Amico classification. In addition, patients with a high value of ratio 2 tended to be classified into a high risk group by the D'Amico classification. From this, it was suggested that the result of determining prostate carcinoma by the method of the present invention is related to the classification by the D'Amico classification.

«Use of a kit for determining prostate carcinoma»

Use of a kit for determining prostate carcinoma according to the present invention is use of a "kit for determining prostate carcinoma, including:

(1) an affinity substance having an affinity for an α(2,3) glycan; and
(2) an instruction manual that describes a determination procedure including obtaining a ratio 1 between an amount of a free prostate specific antigen and an amount of an α(2,3) free PSA in a sample derived from a subject; obtaining a ratio 2 between the ratio 1 and a volume of the prostate of the subject; and determining prostate carcinoma based on the obtained ratio 2".

Details of the affinity substance with α(2,3) glycan affinity and preferred embodiments and specific examples thereof are as described in the section (1)-2 "Affinity substance with α(2,3) glycan affinity".
The affinity substance with α(2,3) glycan affinity may be in the form of a test solution in a solution state such as a suspension suspended in an appropriate buffer solution, or may be a frozen product or a freeze-dried product.
The kit may further include the anti-PSA antibody according to the present invention (an antibody capable of binding to free PSA and bound PSA or/and an anti-free PSA antibody). Details of preferred embodiments and specific examples thereof are as described in the description of the PSA antibody according to the present invention in the section "2. Method for obtaining ratio 1".
The anti-PSA antibody according to the present invention may be in the form of a test solution in a solution state such as a suspension suspended in an appropriate buffer solution, or may be a frozen product or a freeze-dried product.
In a case where the affinity substance with α(2,3) glycan affinity is in the form of a test solution, the anti-PSA antibody according to the present invention may be allowed to coexist in a test solution containing an affinity substance with α(2,3) glycan affinity, or may be contained as a test solution, a frozen product, or a freeze-dried product different from that of the affinity substance with α(2,3) glycan affinity.
The concentration of the affinity substance with α(2,3) glycan affinity and the anti-PSA antibody according to the present invention in the test solution may be any concentration at which the reaction for the desired measurement is started at the time of mixing each test solution. Specifically, it is as described in the section "«Method for determining prostate carcinoma»". In addition, specific examples of the solvent constituting the test solution are also as described in the section "«Method for determining prostate carcinoma»".
The test solution containing the affinity substance with α(2,3) glycan affinity constituting the kit according to the present invention or/and the anti-PSA antibody according to the present invention may include additives commonly used in the related art, for example, reagents, buffering agents, reaction accelerators, saccharides, proteins, salts, stabilizers such as surfactants, and preservatives, which do not inhibit the reaction between α(2,3) free PSA and the affinity substance with α(2,3) glycan affinity. In addition, the concentrations of these reagents may be appropriately selected from the concentration ranges commonly used in the related art.
Hereinafter, the present invention will be described in more detail with reference to Examples and the like, but the present invention is not limited in any way thereby.

Examples

Example 1. Comparison of ratios obtained using samples derived from patients with prostate carcinoma and patients with benign prostatic hyperplasia

(1) Background of patients and collection of samples

Sera collected from 174 patients diagnosed with prostate carcinoma (PCa) and 94 patients diagnosed with non-cancer and benign prostatic hyperplasia (BPH) were used as samples. A biopsy of the prostate of each patient was carried out for histopathological diagnosis.
Table 1 shows the background of the patients (age, total PSA value, and histopathological malignancy classification (biopsy Gleason score, postoperative Gleason score, and the like etc.)).

[Table 1]

| n=268 | BPH (n=94) | | Pca (n=174) | | *p* value |
|---|---|---|---|---|---|
| | median | (range) | median | (range) | |
| Age, (IQR) | 67 | (63-72) | 67 | (63-72) | >0.9999 |

(continued)

| n=268 | BPH (n=94) | | Pca (n=174) | | *p* value |
|---|---|---|---|---|---|
| Prostate volume | 46.7 | (18.6-102.8) | 28.2 | (7.7-94.0) | <0.0001 |
| Total PSA value (ng/mL) | 7.23 | (1.69-26.10) | 7.58 | (1.40-41.23) | 0.1500 |
| PSAD (ng/ml/$cm^3$) | 0.15 | (0.05-0.77) | 0.28 | (0.03-1.62) | <0.0001 |
| Free PSA value/Total PSA value | 0.28 | (0.07-0.75) | 0.17 | (0.00-0.96) | <0.0001 |
| Ratio 1 (%) | 39.1 | (21.8-59.1) | 48.0 | (28.7-100) | <0.0001 |
| Ratio 2 (%/$cm^3$) | 0.81 | (0.32-2.36) | 1.64 | (0.35-6.58) | <0.0001 |
| Prostate biopsy GS | | | n=174 | (%) | |
| GS 3+3 | | | 33 | (18.9) | |
| GS 3+4 | | | 57 | (32.8) | |
| GS 4+3 | | | 18 | (10.3) | |
| GS 4+4, GS 3+5 | | | 26 | (14.9) | |
| GS 4+5, GS 5+4 | | | 40 | (22.9) | |
| Pathological GS | | | n=125 | (%) | |
| GS 3+3 | | | 13 | (10.4) | |
| GS 3+4 | | | 32 | (25.6) | |
| GS 4+3 | | | 19 | (15.2) | |
| GS 4+4, GS 3+5 | | | 13 | (10.4) | |
| GS 4+5, GS 5+4, GS 5+5 | | | 48 | (38.4) | |

PSAD: total PSA value/volume of prostate
Prostate biopsy GS: prostate biopsy Gleason score
Pathological GS: Gleason score after PR

(2) Measurement of volume of prostate

The volume of the prostate of each patient was obtained by ultrasound diagnostic measurements. A general-purpose ultrasound diagnostic imaging apparatus ProSound α7 (Hitachi Aloka Medical, Ltd.) was used for diagnosis, and the volume ($cm^3$) of the prostate was obtained by the software attached to ProSound α4.

(3) Measurement of total PSA

The total PSA value in each sample was obtained using Architect™ Total PSAAbbott (available from Abbott Japan Co., Ltd.), which is an in vitro diagnostic, according to the protocol attached to the kit.

(4) Capillary electrophoresis

(i) Preparation of DNA-labeled anti-PSA antibody

According to the procedure shown in Fig. 2, a DNA-bound PSA antibody Fab' fragment was prepared.
That is, first, a 250 bp DNA fragment introduced with an $NH_2$ group in the 5' terminal was purified by a conventional method (purified terminally-aminated DNA), subsequently the $NH_2$ group introduced in this DNA fragment was reacted with a succinimide group of a sulfosuccinimidyl 4-(p-maleimidephenyl)butyrate (Sulfo-SMPB) linker (a linker having a succinimide group and a maleimide group, manufactured by Pierce Biotechnology, Inc.) by a conventional method, and then the unreacted linker was removed by subjecting the reaction solution to a gel filtration treatment, to obtain a linker-bound 250 bp DNA fragment. The obtained linker-bound 250 bp DNA fragment was reacted with an anti-PSA antibody PSA10 Fab' fragment prepared using an anti-human PSA mouse monoclonal antibody PSA10 (anti-PSA monoclonal antibody clone No. PSA10, manufactured by FUJIFII,M Wako Pure Chemical Corporation) according to a conventional method in advance. The obtained reaction product was purified using a DEAE column to prepare an anti-PSA antibody PSA10 Fab' fragment to which a 250 bp DNA fragment was bound (hereinafter, abbreviated as "DNA-labeled anti-PSA antibody").

It should be noted that the anti-human PSA mouse monoclonal antibody (anti-PSA monoclonal antibody clone No. PSA10) used is an antibody having an affinity for human PSA and binds to bound PSA and free PSA. That is, the antibody binds to α(2,3) free PSA and free PSA other than α(2,3) free PSA.

(ii) Preparation of fluorescently labeled anti-free PSA antibody

An anti-human PSA monoclonal antibody PSA12 which recognizes an epitope of PSA different from an epitope which an anti-PSA monoclonal antibody PSA10 recognizes and specifically binds only to free PSA (anti-PSA monoclonal antibody clone No. PSA12, manufactured by FUJIFII,M Wako Pure Chemical Corporation) was treated by a conventional method to obtain an anti-PSA antibody PSA12 Fab' fragment. A fluorescent substance HiLyte 647 (manufactured by AnaSpec, Inc.) was introduced into an amino group of the resulting fragment by a conventional method, whereby a HiLyte 647-labeled anti-free PSA antibody PSA12 Fab' fragment (hereinafter, abbreviated as "fluorescently labeled anti-free PSA antibody") was obtained.

(iii) Preparation of samples and test solutions

· Preparation of electrophoresis sample A

To a 0.5 mL tube, 2 μL of the sample, 1 μL of the 1 μM fluorescently labeled anti-free PSA antibody prepared in the section (ii), and 7 μL of electrophoresis buffer solution 1 [containing 5% (w/v) polyethylene glycol (PEG20000), 3% (w/v) glycerol, 150 mM NaCl, 0.01% BSA, 75 mM Tris-HCl (pH 7.5), and 10 mM MES] were added and mixed to prepare 10 μL of a reaction solution.
The reaction solution (10 μL) containing the [fluorescently labeled anti-free PSA antibody-free PSA] complex obtained by the reaction was used as electrophoresis sample A.
It should be noted that the final concentration of the fluorescently labeled anti-free PSA antibody in this reaction solution is 100 nM.

· Preparation of electrophoresis buffer solution 2 (containing MAA)

A 75 mM Tris-HCl buffer (pH 7.5) containing 4.5% (w/v) polyethylene glycol (PEG8000), 3% (w/v) glycerol, 10 mM NaCl, and 0.01% BSA was prepared. MAA (manufactured by VECTOR Co., Ltd.) was added thereto to a final concentration of 4 mg/mL and mixed to prepare electrophoresis buffer solution 2.

· Preparation of electrophoresis buffer solution 3

A buffer (pH not adjusted) containing 2% (w/v) polyethylene glycol (PEG20000), 3% (w/v) glycerol, 0.01% BSA, 125 mM HEPES, and 75 mM Tris-HCl was used as electrophoresis buffer solution 3.

· Preparation of electrophoresis buffer solution 4

A 75 mM Tris-HCl buffer (pH 7.5) containing 2% (w/v) polyethylene glycol (PEG20000), 3% (w/v) glycerol, and 0.01% BSA was used as electrophoresis buffer solution 4.

· Preparation of DNA-labeled antibody solution (containing DNA-labeled anti-PSA antibody)

A buffer [containing 2% (w/v) polyethylene glycol (PEG20000), 0.5 mM EDTA(2Na), 3% (w/v) glycerol, 50 mM NaCl, 0.01% BSA, and 75 mM BisTris (pH 6.0)] containing 100 nM of the DNA-labeled anti-PSA antibody obtained in the section (i) was prepared as a DNA-labeled antibody solution.

· Preparation of fluorescent liquid

As the fluorescent liquid, 30 nM HiLyte 647, 50 mM BisTris (pH 6.0) containing 20% (w/v) glycerol was used. The fluorescent liquid is used for adjustment such as position confirmation at the detection unit of the measurement device (μTAS Wako i30).

(iv) Electrophoresis

Microchip capillary electrophoresis was carried out using a fully automated fluorescence immunoassay system

μTAS Wako i30 (manufactured by FUJIFII,M Wako Pure Chemical Corporation) according to the instruction manual of the apparatus by the following procedure.

That is, 5.4 μL of the electrophoresis sample A prepared in the section (iii) was dispensed into predetermined wells (SP wells) of μTAS Wako i30 dedicated microchip. Then, each of the test solutions prepared in the section (iii) was dispensed to each well of the microchip as described below.

· R2 well (R2(FLB) well, R2(LB) well): 10.0 μL of electrophoresis buffer solution 2 (containing MAA),
· R3 well: 10.0 μL of electrophoresis buffer solution 3,
· R4 well: 5.4 μL of electrophoresis buffer solution 4,
· C1 well: 3.0 μL of DNA-labeled antibody solution, and
· FD well: 7.0 μL of fluorescent liquid.

A schematic diagram of the microchip used is shown in Fig. 3.

In Fig. 3, the Waste well is used as a waste reservoir (drain well) at the time of introducing the test solution of each well (R2, R3, R4, and C1) and the electrophoresis sample A into an analysis flow channel.

Subsequently, a pressure of -5 psi was applied in all of the four Waste wells (drain wells) for 30 seconds to introduce the electrophoresis sample A and each test solution into the analysis flow channel of the chip.

Further, after introducing the electrophoresis sample A and each test solution into the analysis flow channel of the chip, PSA was separated and detected by the following method.

Fig. 4 schematically shows an in-chip flow channel of the microchip used.

In Fig. 4, W represents a Waste well. The R3 well side serves as a negative electrode and the R2(LB) well side serves as a positive electrode. In addition, in Fig. 4, the placement portion of the electrophoresis sample A and the test solution in each well is indicated by coloring it into a dot portion and a white portion (a portion without a dot).

A voltage of 4000 V was applied between the R3 well and the R2(LB) well in Fig. 4, and the DNA-labeled anti-PSA antibody in the test solution was brought into contact with the [fluorescently labeled anti-free PSA antibody-free PSA] complex in the electrophoresis sample A at 30°C to produce a [fluorescently labeled anti-free PSA antibody-free PSA-DNA-labeled anti-PSA antibody] complex, which was then concentrated by isotachophoresis (ITP). The electrophoresis direction of isotachophoresis is indicated by "ITP" and dotted line in Fig. 4.

The immune reaction time with each labeled antibody to capture free PSA was about 200 seconds.

Specifically, the complexes formed here are a [fluorescently labeled anti-free PSA antibody-α(2,3) free PSA-DNA-labeled anti-PSA antibody] complex (first complex) and a [fluorescently labeled anti-free PSA antibody-free PSA other than α(2,3) free PSA-DNA-labeled anti-PSA antibody] complex (second complex).

After the complex was subjected to isotachophoresis to the R2(FLB) well and its passing through the R2(FLB) well was determined from a change in voltage, a negative electrode was switched from R3 to R2(FLB). Then, the capillary gel electrophoresis (CE) was further carried out in the presence of MAA until a peak of the [fluorescently labeled anti-free PSA antibody-free PSA-DNA-labeled anti-PSA antibody] complex was detected in the detection portion (a capillary portion 2 cm downstream from the channel crossing portion of R2(FLB) and R2(LB)). The position where CE was carried out and the electrophoresis direction of electrophoresis are indicated by "CE" and dotted line in Fig. 4.

It should be noted that the detection was carried out by measuring over time an intensity of fluorescence generated by 635 nm laser excitation in the capillary portion 2 cm downstream from the channel crossing portion of R2(FLB) and R2(LB), using a photodiode (manufactured by FUJIFILM Corporation).

In addition, PSA was separated and detected in such a manner that the same method as described above was carried out using the same electrophoresis sample A and electrophoresis test solution and measurement device as described above, except that electrophoresis buffer solution 2 not containing MAA was used.

The peak of the second complex appears at the same position as that of the peak that appears in a case where the electrophoresis buffer solution 2 containing no MAA is used. On the other hand, since the [fluorescently labeled anti-free PSA antibody-α(2,3) free PSA-DNA-labeled anti-PSA antibody] complex (first complex) which reacts with MAA takes time to migrate as compared with the [fluorescently labeled anti-free PSA antibody-free PSA other than α(2,3) free PSA-DNA-labeled anti-PSA antibody] complex (second complex) which does not react with MAA, the appearance of the peak is delayed. That is, the peak of the first complex appears after the peak of the second complex.

The peak area of the fraction of the first complex and the peak area of the fraction of the second complex thus obtained were obtained by the analysis software attached to the measurement device.

Then, using the peak area of the fraction of the first complex and the peak area of the fraction of the second complex thus obtained, the ratio (%) of the amount of α(2,3) free PSA to the amount of free PSA in the sample was calculated.

A specific calculation method is as follows.

· Amount of free PSA = [peak area of fraction of first complex] + [peak area of fraction of second complex]

.

$$\cdot \text{ Ratio (\%) of amount of } \alpha(2,3) \text{ free PSA to amount of free PSA (ratio 1)}$$
$$= [\text{peak area of fraction of first complex}]/[\text{amount of free PSA}] \times 100$$

Then, the ratio of the ratio 1 of each patient obtained above to the prostate volume of the same patient (ratio 2) was obtained.

.

$$\cdot \text{ Ratio 2: Ratio (\%) of ratio 1 to prostate volume}$$
$$= [(\text{peak area of fraction of first complex}/\text{amount of free PSA}) \times 100]/\text{prostate volume}$$
$$= \text{ratio 1 (\%)}/\text{prostate volume}$$

Table 1 shows the results obtained for prostate carcinoma patients (PCa) and benign prostatic hyperplasia patients (BPH). In Table 1, the average value ($cm^3$) of volume of prostate, the average value (%) of ratio 1, and the average value (%/$cm^3$) of ratio 2 are shown.
Based on the above results, a Relative Operating Characteristic curve (ROC curve) analysis was further carried out.

(5) Results

The results are shown in Fig. 5.
In Fig. 5, (1) and (2) are ROC curves created based on the ratio 2. (3) and (4) are ROC curves created based on the ratio 1.
In addition, in Fig. 5, (1) and (3) show the results in a case of using a serum derived from a subject having a total PSA value in serum of 50 ng/mL or less. (2) and (4) show the results in a case of using a serum derived from a subject having a total PSA value in serum of 10 ng/mL or less.
Then, according to a known method, the biopsy avoidance rate was obtained from the value of specificity at a diagnostic sensitivity of 90%. In Fig. 5, the lateral axis indicates [100 - specificity]. Therefore, from Fig. 5, [100 - value on lateral axis at 90% diagnostic sensitivity] = specificity (%) at 90% diagnostic sensitivity = biopsy avoidance rate (%).
The results obtained by the ROC curve analysis are summarized in Table 2 below.

[Table 2]

| | Ratio 2 | | Ratio 1 | |
|---|---|---|---|---|
| Total PSA | 50 ng/mL or less | 10 ng/mL or less | 50 ng/mL or less | 10 ng/mL or less |
| AUC | 0.83 | 0.82 | 0.79 | 0.77 |
| Biopsy avoidance rate | 55% | 55% | 40% | 40% |

As is clear from Fig. 5 and Table 2, an Area Under the Curve (AUC) in a case of determining prostate carcinoma using the ratio 2 as an index is greater than the AUC in a case of determining prostate carcinoma using the ratio 1 as an index. From this, it can be seen that the determination method using the ratio 2 as an index is superior to the determination method using the ratio 1 as an index in terms of determination sensitivity and specificity.
Table 3 below summarizes the results of ROC curve analysis in a case where a sample with a total PSA value of 10 ng/mL or less was used (case (2) in Fig. 5).

[Table 3]

| Biomarker | Ratio 2 | Ratio 1 | Total PSA | PCA3 | PHI |
|---|---|---|---|---|---|
| Cutoff | 0.895 | 40% | 4 | 20-35 | - |
| AUC | 0.82 | 0.77 | 0.51 | 0.66-0.69 | 0.70-0.77 |

(continued)

| Biomarker | Ratio 2 | Ratio 1 | Total PSA | PCA3 | PHI |
|---|---|---|---|---|---|
| PPV (%) | 78 | 73 | 65 | - | - |
| NPV (%) | 78 | 69 | 55 | 88-90 | 67-92 |
| Risk of missing Pca (%) | 10 | 10 | 10 | 10-12 | 8-33 |
| Avoided Biopsies (%) | 55 | 36 | 20 | 44 | 36 |

In Table 3, according to the usual method of obtaining a cutoff value using the ROC curve, the cutoff value was obtained in such a manner that a straight line with an angle of 45 degrees in contact with the ROC curve of Fig. 5 is drawn, and a value of an intersection point with that straight line, that is, a value at which maximizes the "sensitivity - (100% - specificity %)" is obtained.

As is clear from Table 3, the biopsy avoidance rate (Avoided Biopsies (%)) in a case of using the ratio 2 was 55%. On the other hand, the biopsy avoidance rate in a case of using the ratio 1 was 36%.

From the above, it was demonstrated that the method for determining prostate carcinoma according to the present invention using the ratio 2 has a higher biopsy avoidance rate and a larger AUC than the method for determining prostate carcinoma using the ratio 1.

In addition, in the verification of the present example, the cutoff value in a case of determining prostate carcinoma based on the ratio 1 was "40%". In addition, the cutoff value in a case of determining prostate carcinoma based on the ratio 2 was "0.895".

Although data are not shown, the ratio 2 of the prostate carcinoma patients obtained in the present example was compared with the ratio of D'Amico classification to find that patients with a low value of ratio 2 according to the present invention tended to be classified into a low risk group by the D'Amico classification. In addition, patients with a high value of ratio 2 tended to be classified into a high risk group by the D'Amico classification. From this, it was suggested that the result of determining prostate carcinoma by the method of the present invention is related to the classification by the D'Amico classification.

In addition, based on the total PSA value separately obtained as described above, an analysis was similarly carried out using an ROC curve. As a result, the biopsy avoidance rate was 20% in a case where prostate carcinoma was determined based on the total PSA value.

The cutoff value in a case of determining prostate carcinoma based on the total PSA value is 4.0.

However, since the method for determining prostate carcinoma based on the total PSA value has low specificity, there are cases where prostate carcinoma is suspected from other symptoms even in a case where the total PSA value is less than the cutoff value. In that case, even in a case where the total PSA value is less than the cutoff value, most cases are used for definitive diagnosis. Therefore, in actual clinical diagnosis, the biopsy avoidance rate of the method for determining prostate carcinoma based on the total PSA value is considered to be significantly lower than 20%.

In addition, for reference, data of the determination method using PCA3 (prostate cancer antigen 3) as an index and data of the determination method using Prostate Health Index (PHI) as an index are also shown in Table 3.

As the respective data relating to the determination method using PCA3 as an index described in Table 3, the data described in the following literature are reproduced respectively.

Cutoff: Gittelman M et al. J Urol 2013;190:64-9, Wei JT et al. J Clin Oncol 2014;20;32:4066-72, Haese A et al. Eur Urol 2008;54:1081-8.
AUC: Haese A et al. Eur Urol 2008;54:1081-8, Aubin SM et al. J Urol 2010;184:1947-52,
NPV(%): Wei JT et al. J Clin Oncol 2014;20;32:4066-72,
Risk of Pca(%): Gittelman M et al. J Urol 2013;190:64-9, Wei JT et al. J Clin Oncol 2014;20;32:4066-72, Haese A et al. Eur Urol 2008;54 :1081-8, Aubin SM et al. J Urol 2010;184:1947-52, and
Avoided Biopsies (%): Haese A et al. Eur Urol 2008;54:1081-8, Aubin SM et al. J Urol 2010;184:1947-52.

In addition, as the respective data relating to the determination method using PHI as an index described in Table 3, the data described in the following literature are reproduced respectively.

AUC: Filella X et al. Clin Chem Lab Med 2013;51:729-39,
PPV(%): Filella X et al. Clin Chem Lab Med 2013;51:729-39,
NPV(%): Filella X et al. Clin Chem Lab Med 2013;51:729-39,
Risk of missing Pca(%): Filella X et al. Clin Chem Lab Med 2013;51:729-39, De la Calle C et al. J Urol 2015;194:65-72, and

Avoided Biopsies (%): Filella X et al. Clin Chem Lab Med 2013;51:729-39.

As is clear from Table 3, it was found that the method of the present invention for determining prostate carcinoma using the ratio 2 has a biopsy avoidance rate and an AUC greater than those of the conventional determination method using PCA3 as an index and the conventional determination method using PHI as an index.
From the above, it was found that the method for determining prostate carcinoma according to the present invention can determine prostate carcinoma with higher accuracy and with higher biopsy avoidance rate than the conventional determination methods.
Therefore, the method of the present invention can greatly contribute to reducing the risk of suffering a disadvantage due to an unnecessary biopsy, that is, overdiagnosis, in a case suspected of having prostate carcinoma based on a conventional serum marker (total PSA value).

Industrial Applicability

The method for determining prostate carcinoma according to the present invention can determine prostate carcinoma with a high biopsy avoidance rate, and therefore has a significant effect of avoiding the problem that a subject undergoes an excessive biopsy.

## Claims

**1.** A method for determining prostate carcinoma, comprising:

measuring an amount of a free prostate specific antigen and an amount of a free PSA having an α(2,3) glycan, the free PSA having an α(2,3) glycan being a free prostate specific antigen having a glycan in which a terminal sialic acid residue of the glycan is α(2,3)-linked to a second galactose residue from a terminal of the glycan, in a sample derived from a subject;
obtaining a ratio 1 between the amount of the free prostate specific antigen and the amount of the free PSA having an α(2,3) glycan;
obtaining a ratio 2 between the ratio 1 and a volume of the prostate of the subject; and
determining prostate carcinoma based on the obtained ratio 2.

**2.** The method for determining prostate carcinoma according to claim 1, wherein a method for measuring the amount of the free PSA having an α(2,3) glycan is a method including:

reacting the free PSA having an α(2,3) glycan with an affinity substance having an affinity for an α(2,3) glycan, the affinity substance being a substance having an affinity for a glycan in which a terminal sialic acid residue of the glycan is α(2,3)-linked to a second galactose residue from the terminal of the glycan, to form a complex of the free PSA having an α(2,3) glycan and the affinity substance having an affinity for an α(2,3) glycan;
measuring an amount of the complex; and
obtaining an amount of the free PSA having an α(2,3) glycan based on obtained measurement results.

**3.** The method for determining prostate carcinoma according to claim 2, wherein the affinity substance having an affinity for an α(2,3) glycan is a lectin.

**4.** The method for determining prostate carcinoma according to claim 3, wherein the lectin is a *Maackia amurensis* lectin.

**5.** A method for obtaining data for carrying out a prostate carcinoma determination, comprising:

obtaining a ratio 1 between an amount of a free prostate specific antigen and an amount of a free PSA having an α(2,3) glycan in a sample derived from a subject according to the methods of claims 1-4; and
obtaining a ratio 2 between the ratio 1 and a volume of the prostate of the subject.

**6.** Use of a kit for determining prostate carcinoma, comprising:

(1) an affinity substance having an affinity for an α(2,3) glycan; and
(2) an instruction manual that describes a determination procedure including obtaining a ratio 1 between an amount of a free prostate specific antigen and an amount of a free PSA having an α(2,3) glycan in a sample

derived from a subject; obtaining a ratio 2 between the ratio 1 and a volume of the prostate of the subject; and determining prostate carcinoma based on the obtained ratio 2.

## Patentansprüche

**1.** Verfahren zum Bestimmen eines Prostatakarzinoms, umfassend:

Messen einer Menge eines freien prostataspezifischen Antigens und einer Menge eines freien PSA mit einem α(2,3)-Glykan in einer von einem Individuum stammenden Probe, wobei das freie PSA mit einem α(2,3)-Glykan ein freies prostataspezifisches Antigen ist, das ein Glykan aufweist, in welchem ein endständiger Sialinsäurerest des Glykans mit einem zweiten Galaktoserest von einem Ende des Glykans α(2,3)-verknüpft ist;
Erhalten eines Verhältnisses 1 zwischen der Menge des freien prostataspezifischen Antigens und der Menge des freien PSA mit einem α(2,3)-Glykan;
Erhalten eines Verhältnisses 2 zwischen dem Verhältnis 1 und einem Volumen der Prostata des Individuums; und Bestimmen des Prostatakarzinoms auf der Grundlage des erhaltenen Verhältnisses 2.

**2.** Verfahren zum Bestimmen eines Prostatakarzinoms nach Anspruch 1, wobei ein Verfahren zum Messen der Menge des freien PSA mit einem α(2,3)-Glykan, ein Verfahren ist, das Folgendes umfasst:

Umsetzen des freien PSA mit einem α(2,3)-Glykan mit einer Affinitätssubstanz mit einer Affinität für ein α(2,3)-Glykan, wobei die Affinitätssubstanz eine Substanz ist, die eine Affinität für ein Glykan aufweist, in welchem ein endständiger Sialinsäurerest des Glykans mit einem zweiten Galaktoserest von einem Ende des Glykans α(2,3)-verknüpft ist, um einen Komplex aus dem freien PSA mit einem α(2,3)-Glykan und der Affinitätssubstanz mit einer Affinität für ein α(2,3)-Glykan zu bilden;
Messen einer Menge des Komplexes; und
Erhalten einer Menge des freien PSA mit einem α(2,3)-Glykan auf der Grundlage der erhaltenen Messergebnisse.

**3.** Verfahren zum Bestimmen eines Prostatakarzinoms nach Anspruch 2, wobei die Affinitätssubstanz mit einer Affinität für ein α(2,3)-Glykan ein Lektin ist.

**4.** Verfahren zum Bestimmen eines Prostatakarzinoms nach Anspruch 3, wobei das Lektin ein *Maackia amurensis* Lektin ist.

**5.** Verfahren zum Erhalt von Daten für die Durchführung einer Prostatakarzinombestimmung, umfassend:

Erhalten eines Verhältnisses 1 zwischen einer Menge eines freien prostataspezifischen Antigens und einer Menge eines freien PSA mit einem α(2,3)-Glykan in einer von einem Individuum stammenden Probe gemäß den Verfahren der Ansprüche 1-4; und
Erhalten eines Verhältnisses 2 zwischen dem Verhältnis 1 und einem Volumen der Prostata des Individuums.

**6.** Verwendung eines Kits zum Bestimmen eines Prostatakarzinoms, welches umfasst:

(1) eine Affinitätssubstanz mit einer Affinität für ein α(2,3)-Glykan; und
(2) eine Gebrauchsanweisung, die ein Bestimmungsverfahren beschreibt mit Erhalten eines Verhältnisses 1 zwischen einer Menge eines freien prostataspezifischen Antigens und einer Menge eines freien PSA mit einem α(2,3)-Glykan in einer von einem Individuum stammenden Probe; Erhalten eines Verhältnisses 2 zwischen dem Verhältnis 1 und einem Volumen der Prostata des Individuums; und Bestimmen eines Prostatakarzinoms auf der Grundlage des erhaltenen Verhältnisses 2.

## Revendications

**1.** Procédé de détermination d'un carcinome de la prostate, comprenant les étapes consistant à :

mesurer une quantité d'un antigène prostatique spécifique libre et une quantité d'un APS libre présentant un glycane a(2,3), l'APS libre présentant un glycane α(2,3) étant un antigène prostatique spécifique libre présentant

un glycane dans lequel un résidu d'acide sialique terminal du glycane est lié par α(2,3) à un second résidu de galactose à partir d'une extrémité du glycane, dans un échantillon dérivé d'un sujet ;
obtenir un rapport 1 entre la quantité de l'antigène prostatique spécifique libre et la quantité de l'APS libre présentant un glycane α(2,3) ;
obtenir un rapport 2 entre le rapport 1 et un volume de la prostate du sujet ; et
déterminer un carcinome de la prostate sur la base du rapport 2 obtenu.

**2.** Procédé de détermination d'un carcinome de la prostate selon la revendication 1, dans lequel un procédé de mesure de la quantité de l'APS libre présentant un glycane α(2,3) est un procédé incluant les étapes consistant à :

faire réagir l'APS libre présentant un glycane α(2,3) avec une substance d'affinité présentant une affinité pour un glycane a(2,3), la substance d'affinité étant une substance présentant une affinité pour un glycane dans lequel un résidu d'acide sialique terminal du glycane est lié par a(2, 3) à un second résidu de galactose à partir de l'extrémité du glycane, pour former un complexe de l'APS libre présentant un glycane α(2,3) et de la substance d'affinité présentant une affinité pour un glycane α(2,3) ;
mesurer une quantité du complexe ; et
obtenir une quantité de l'APS libre présentant un glycane α(2,3) sur la base des résultats de mesure obtenus.

**3.** Procédé de détermination d'un carcinome de la prostate selon la revendication 2, dans lequel la substance d'affinité présentant une affinité pour un glycane α(2,3) est une lectine.

**4.** Procédé de détermination d'un carcinome de la prostate selon la revendication 3, dans lequel la lectine est une lectine de Maackia amurensis.

**5.** Procédé d'obtention de données pour exécuter une détermination d'un carcinome de la prostate, comprenant les étapes consistant à :

obtenir un rapport 1 entre une quantité d'un antigène prostatique spécifique libre et une quantité d'un APS libre présentant un glycane α(2,3) dans un échantillon dérivé d'un sujet selon les procédés des revendications 1-4 ; et
obtenir un rapport 2 entre le rapport 1 et un volume de la prostate du sujet.

**6.** Utilisation d'un kit pour déterminer un carcinome de la prostate, comprenant :

(1) une substance d'affinité présentant une affinité pour un glycane α(2,3) ; et
(2) un manuel d'instruction qui décrit une procédure de détermination incluant les étapes consistant à obtenir un rapport 1 entre une quantité d'un antigène prostatique spécifique libre et une quantité d'un APS libre présentant un glycane α(2,3) dans un échantillon dérivé d'un sujet ; obtenir un rapport 2 entre le rapport 1 et un volume de la prostate du sujet ; et déterminer un carcinome de la prostate sur la base du rapport 2 obtenu.

FIG. 1

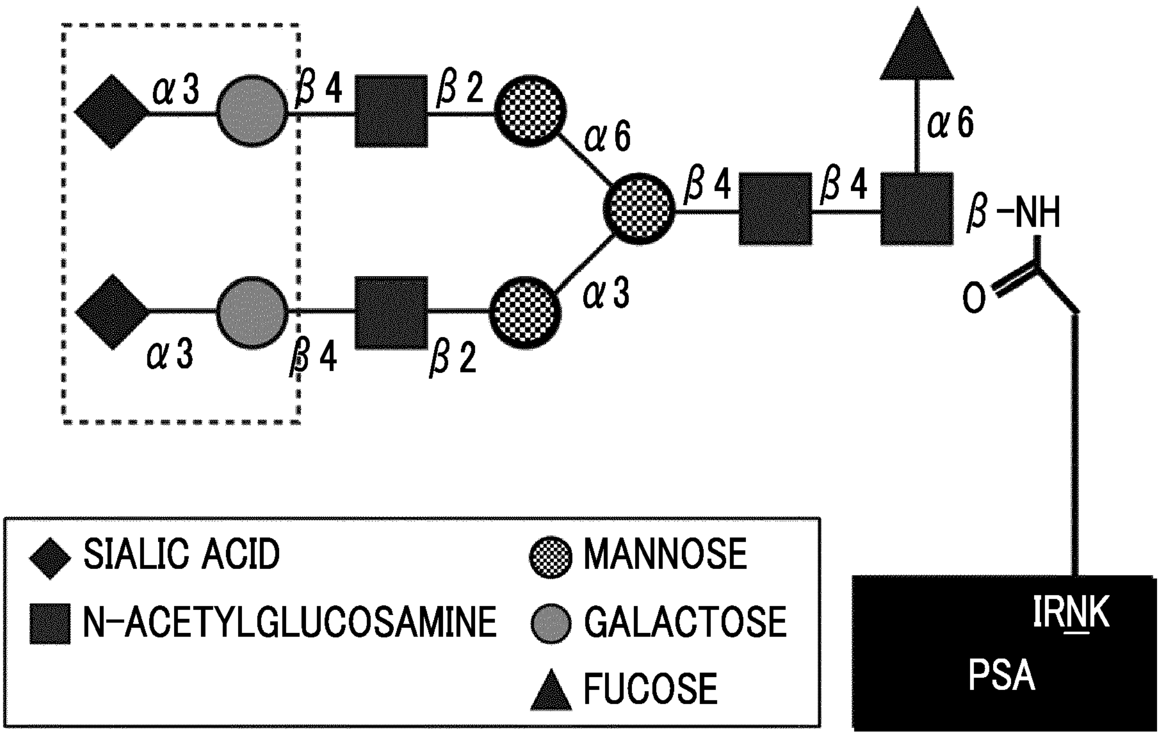
α3
β4
β2
α6
β4
β4
α6
β-NH
O
α3
α3
β4
β2
SIALIC ACID
N-ACETYLGLUCOSAMINE
MANNOSE
GALACTOSE
FUCOSE
IRNK
PSA

FIG. 2

PURIFIED TERMINALLY-AMINATED DNA

← Sulfo-SMPB LINKER REACTION

← REMOVAL OF UNREACTED LINKER BY GEL FILTRATION

← REACTION WITH ANTI-PSA ANTIBODY PSA10 Fab' FRAGMENT

← PURIFICATION OF DNA-LABELED ANTI-PSA ANTIBODY BY DEAE COLUMN

↓

DNA-LABELED ANTI-PSA ANTIBODY

FIG. 3

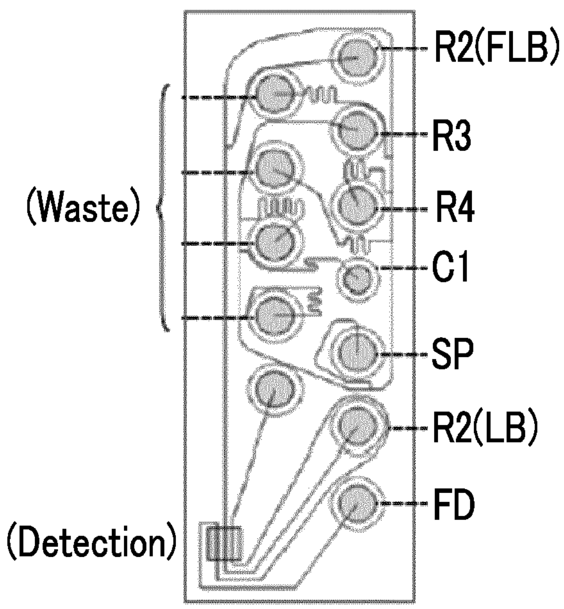

FIG. 4

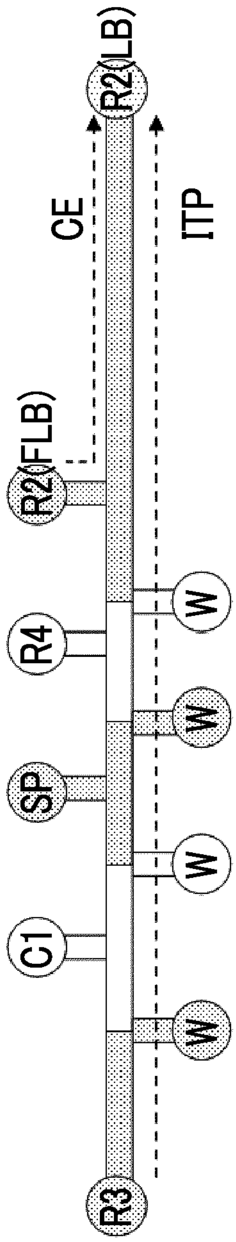

FIG. 5

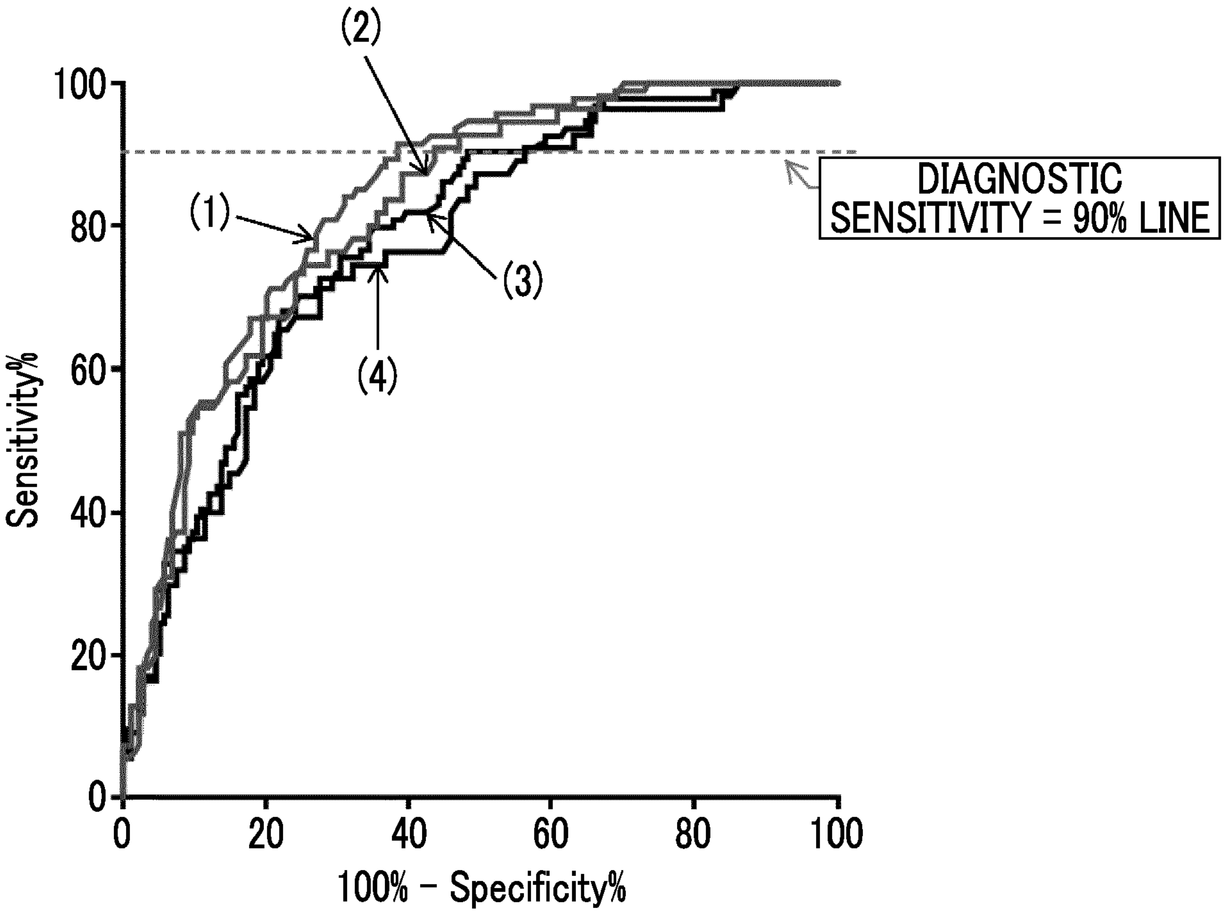
(1)
(2)
(3)
(4)
100
80
60
40
20
0
Sensitivity%
0
20
40
60
80
100
100% - Specificity%
DIAGNOSTIC
SENSITIVITY = 90% LINE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017130578 A
- EP 2908136 A1
- JP 4214779 B
- JP 4862093 B
- WO 2007027495 A


### Non-patent literature cited in the description


- **STAMEY T. A. et al.** *N. Engl. J. Med.,* 1987, vol. 317, 909-916
- **TAJIRI M. ; OHYAMA C. ; WADA Y.** *Glycobiolgy,* 2008, vol. 18, 2-8
- **SUZUKI H. et al.** *Urology,* 2006, vol. 67 (1), 131-136
- **TOMOKAZU ISHIKAWA et al.** *International Journal of Molecular Sciences,* February 2017, vol. 18 (2), 470
- **KAZUTO ITO et al.** Cancer. American Cancer Society, 31 October 2022, vol. 95, 2112-2119
- *Eur. J. Immunol.,* 1976, vol. 6, 511
- **CHOKU MATSUHASHI et al.** Introduction to Immunology Experiments. Academic Publishing Center, 1981
- Medical Chemistry Experimental Course. Nakayama Shoten Co., Ltd, 1971, vol. 8
- **AKIRA KAWAOI.** Illustrative Fluorescent Antibodies. Soft Science Inc, 1983
- Enzyme Immunoassays. Igaku-Shoin Ltd, 1982
- Enzyme Immunoassays. Proteins, Nucleic Acids, and Enzymes. Kyoritsu Shuppan Co., Ltd, 1987, vol. 31, 51-63
- Enzyme Immunoassays. Proteins, Nucleic Acids, and Enzymes. Kyoritsu Shuppan Co., Ltd, 1987, vol. 31, 252-263
- Enzyme Immunoassays. Proteins, Nucleic Acids, and Enzymes. Kyoritsu Shuppan Co., Ltd, 1987, vol. 31, 264-271
- **YONEYAMA T. et al.** *Biochem Biophys Res Commun.,* 2014, vol. 448 (4), 399-396
- **J. SAMBROOK ; E. F. FRITSCH ; T. MANIATIS.** Molecular Cloning: A Laboratory Manual. Cold Spring Harbor Laboratory Press
- *J. Chromatogr.,* 1992, vol. 593, 253-258
- *Anal. Chem.,* 1992, vol. 64, 1926-1932
- Chapter 9: Lectin Microarrays. **MASAO YAMADA.** MICROARRAY METHODS AND PROTOCOLS. CRC Press, 2009, 141
- **KUNO A. et al.** *Nat Methods.,* November 2005, vol. 2 (11), 851-856
- **GITTELMAN M et al.** *J Urol,* 2013, vol. 190, 64-9
- **WEI JT et al.** *J Clin Oncol,* 2014, vol. 20 (32), 4066-72
- **HAESE A et al.** *Eur Urol,* 2008, vol. 54, 1081-8
- **AUBIN SM et al.** *J Urol,* 2010, vol. 184, 1947-52
- **FILELLA X et al.** *Clin Chem Lab Med,* 2013, vol. 51, 729-39
- **DE LA CALLE C et al.** *J Urol,* 2015, vol. 194, 65-72